# EUROPEAN PATENT APPLICATION

(11) **EP 2 426 202 A1**
(43) Date of publication of application: **07.03.2012**
(21) Application number: 10075383.9
(22) Date of filing: 03.09.2010
(51) Int. Cl.: C12N 15/113, A61K 31/00

(54) **Kinases as targets for anti-diabetic therapy**

(71) Applicant: Max-Planck-Gesellschaft zur Förderung der Wissenschaften e.V., 80539 München (DE)
(72) Inventor: Bäcker, Mathias, 82152 Martinsried (DE); Ullrich, Axel, 80331 Munich (DE)
(74) Representative: Arth, Hans-Lothar

(57) **Abstract**

The present invention is related to compound capable of modulating the activity and/or expression of the protein kinases SCYL1, ADCK1, GRK5 and CSNK2A1, thereby enhancing the expression and/or release of insulin. The invention is further related to methods of identifying said compounds for the treatment of metabolic diseases. The invention is further related to methods of treatment of metabolic diseases, particularly diabetes mellitus type 2.

SEQUENCE LISTING

<110>  Max-Planck-Gesellschaft zur Förderung der Wissenschaften

<120>  Kinases as targets for anti-diabetic therapy

<130>  ULA-P03213EP

<160>  16

<170>  PatentIn version 3.3

<210>  1
<211>  23
<212>  DNA
<213>  Mus musculus

<400>  1
cggcggcgac gatgtggttc ttt                                              23


<210>  2
<211>  22
<212>  DNA
<213>  Mus musculus

<400>  2
cggcgttgcc ctgtgccgag ta                                               22


<210>  3
<211>  23
<212>  DNA
<213>  Mus musculus

<400>  3
ctgacacggg caaggctgag att                                              23


<210>  4
<211>  23
<212>  DNA
<213>  Mus musculus

<400>  4
gcgccctgat acaacaccga gac                                              23


<210>  5
<211>  22
<212>  DNA
<213>  Mus musculus

<400>  5
gccgggtgct ggagactgag ga                                               22


<210>  6
<211>  24
<212>  DNA
<213>  Mus musculus

<400>  6
tggcggttct ggaggctgac ttct                                             24


<210>  7
<211>  24

<212>  DNA
<213>  Mus musculus

<400>  7
ggctggccac ttcatctctt ctct                                              24


<210>  8
<211>  21
<212>  DNA
<213>  Mus musculus

<400>  8
tgcggccaca tcacccatta g                                                 21


<210>  9
<211>  2637
<212>  DNA
<213>  Mus musculus

<400>  9
cccgccccgc cccggctcgg gcggccggag gacccggagt ggaggctccc gagcccgtgg      60

cggcggcgac gatgtggttc tttgcccggg acccggtccg ggacttcccg ttcgagctga     120

gccctgagcc ccccgaaggc gggccgcccg ggccctggat cctgcaccga ggccgcaaaa     180

aggccacagg cagcgcagtg tccatcttcg tgtatgatgt gaaaccggga gctgaagagc     240

agacccaggt ggccaaagct gccttcaaac gcctcaaaac tctccgacac cccaacatcc     300

tggcctatat cgatgggttg gagacagaaa agtgcctcca catcgtgaca gaggctgtga     360

cccccctggg aacatacctc aaggcacgag cagaagcagg tggcctgaag gagcaggagc     420

tgtcatgggg gttacaccag atcgtgaaag ccctcagctt cctggtcaac gactgcaacc     480

tcatccacaa taatgtctgc atggccgctg tgtttgtgga cagggctggc gagtggaaac     540

ttgggggtct ggactacatg tactcggcac agggcaacgg cgggggacca cccagcaagg     600

ggatcccgga gctcgagcag tatgatcccc cggagctggc tgacagcagt agcagagcag     660

tcagagagaa gtggtcagca gacatgtggc gcttgggctg cctcatctgg gaagttttca     720

atgggtctct acctcgggca gctgccctgc gcaaccctgg gaagatcccc aaatccctgg     780

tgacccatta ctgtgaactg gtgggagcta acccaaaagt acgtcccaac ccggcccgct     840

tcctgcagaa ctgccgggca cccggtggct tcatgagcaa ccgctttgtt gagaccaacc     900

tcttcctgga ggagattcag atcaaagagc cagctgagaa gcagaagttc ttccaagagc     960

tgagcaagag tctagactca tttcccgaag atttctgtcg acacaaggtg ctgccccagc    1020

tactgactgc ctttgagttt ggcaatgctg gggccgtggt cctcacacct ctcttcaagg    1080

tgggaaaatc cctccgtgct gaagagtacc aggagaagat catccccgtg gtagttaaga    1140

tgttctcatc caccgaccgg gccatgcgca tccgcctcct ccagcagatg gagcagttca    1200

tccaatacct tgatgagcca acagtcaaca cgcagatttt cccccacgtc acacatggct    1260

tcctggacac caaccccgcc atccgcgagc agacggtcaa gtccatgctg ctcttggccc    1320

caaagctgaa tgaggccaat ctcaatgtgg aactgatgaa gcactttgca aggctacaag    1380

ccaaggacga ccagggtcct atccgctgca acaccacggt ctgcttgggc aaaatcggct 1440

cctatctcag tgctagtact agacacaggg tcctcacctc cgccttcagc agagccacta 1500

aggacccatt tgcaccatcc cgggttgcgg gtgtcctggg ctttgctgcc acacacaatc 1560

tctattcgat ggacgactgt gcccataaga tcctgcctgt gctctgtggc cttactgtgg 1620

accctgagaa atctgtgcgg gaccaggcct ttaagaccat tcgaagcttc ctgtccaaat 1680

tagagtctgt gtcagaggat cccacccagc tggcagaagt agagaaggat gtccatgcag 1740

cgtccagtcc tggaacagga ggagctgcag ccagctgggc aggctgggct gtgactgggg 1800

tatcctctct cacctccaag ctgatccgag cacaccccac gcctgtgccg tctgatacca 1860

ctgtgcccca gagaccagtg ccagagggaa atcctgctcc agcccctgcc cttgcccaag 1920

ctatccctgc aacctcaggg cactgggaga cacaggaaga caaggacact gcagaagaca 1980

gcgccactgc tgacagatgg gacgatgagg actggggcag cttggagcag gaagctgaat 2040

ccgtgttggc acagcaggat gactggagtg ccaagggcca aggaagccga gctggacaga 2100

tcaaccaccc agaccacaaa tctctggaat cacattggag cagctgggaa gttgagggct 2160

cctgggacca gggctggcag gaacccagct ctgtggagcc acctccagaa ggcactcggc 2220

tagctagcga atataactgg ggtggtgcag agcccagtga caagggcgac ccctttgctg 2280

ccctgtctgt tcgtcccagc gctcagccca ggccagaccc agactcctgg ggtgaagaca 2340

actgggaagg cttggaggct gagagcagac aggtaaaggc agagctggcc cggaaaaagc 2400

gagaggaaag gagaagagaa atggaagcca aacgggcaga gaaaaagacc accaaggggc 2460

ccatgaagct gggagcccgg aagctggact gacaacccca cccccaagcc actgggcttc 2520

caaccactgg agagcaggcc cggcggatgt atttattgta caaaccatgt gagcctggtc 2580

agcaggtcag gcacatctag tgtacataat cagagccaca ataaattcta tttcaca 2637

<210> 10
<211> 3182
<212> DNA
<213> Mus musculus

<400> 10
gcggccgcca ggtcgccccg ggctggccga accccttgct gcccggaaac aaagtgcacc 60

tttgtcagag acctggcgcg tgtgagcggc agacggcggg ccgcgcggaa ccccaggagc 120

gcgccttcgc tcgggtctca gctcactcct gggcctccca cagcagcgtt ctgccgcctt 180

ggcttgtgct ttccgctttc tcggatcttg gcgggacagg aaagggactc tgcgctccag 240

gagtgggggt ttcgttttgg ctgagtcgtt tttgactcct gccggcgagg ctgggccgcc 300

tgggctcagc gccataccgg cagcagtcct gctccacagt cccggtgcaa tagagccctc 360

gccccgtcag acgcgggact acaattccca gcactcctcg cgttcagagc gcgcggtggg 420

agttgcctcc gggcagacgc ctgcgcgctg cagcggctcg agcctggagt accacacccc 480

cgggagggga acgaggggag gctgaagcat ccgaagcatt aaagcatccg agggagccgg 540

aggggaggag aatggagtga cagagacgcg cggagggtgg ggggtggggg ggaaagtgtt 600

gagggagggg ggagggggga cacagaggga ggaagaagcg gcggcggcgt ctcttcggtg 660
cagaggggga aactccgcgg gctccgagaa agaataatgc ggtagcaggc aggctgcttg 720
ctctggggtt tggcagcagc ggcggcagct cgagcagtgg cagcggcagc ggcatcaccc 780
cagacgctga cagcacagcc ggccggctcc ctcgctgact gccgactgtc aatggagctg 840
gaaaacatcg tggccaacac ggtcttgctg aaagcccggg aagggggtgg aggaaagcgc 900
aaagggaaaa gcaagaagtg gaaggaaatc ctgaagtttc ctcacatcag ccagtgtgaa 960
gacctccgaa ggaccataga cagagattac tacagtctat gtgacaagca accaattggg 1020
agactgcttt ttcgacagtt ctgtgaaacc aggcctgggc tggagtgcta cattcagttc 1080
ctggacttag tggcagaata tgaaattact ccagatgaaa accttggggc gaaggggaag 1140
gaaataatga ccaagtacct cactccaaag tccccagtct tcattgccca agttggacag 1200
gacctggtct cccagacaga gaagaagctc ctgcagagcc cctgcaaaga actcttctct 1260
gcttgtgctc agtctgtcca tgactacttg aagggagacc ccttccacga gtacctggat 1320
agcatgtatt ttgaccgttt tctgcagtgg aaatggttag aaagacaacc agtgaccaaa 1380
aacactttcc ggcagtaccg agtgctgggc aaagggggct ttggagaggt ctgtgcctgc 1440
caggttcggg ccactggtaa aatgtatgct tgtaaacgct tagagaagaa gaggatcaaa 1500
aagaggaaag gcgaatccat ggcactcaac gaaaagcaga ttcttgagaa ggtcaacagc 1560
cagtttgtgg tcaacctggc ctatgcctat gaaaccaaag atgcactatg cctggttctg 1620
accattatga atggtggtga cctgaagttt cacatctaca atatggggaa tcctggcttt 1680
gaggaagagc gagccttatt ttatgcagct gagatcctct gtggcctaga agacttacac 1740
cgtgagaaca ctgtctatag agatctaaaa cccgaaaaca tcttgctgga tgattatggc 1800
cacataagga tctcagacct cggactggcc gtgaagatcc ccgagggaga ccttatccgt 1860
ggccgggtag gcactgttgg ctacatggcc ccagaagttc tgaacaacca gcgatatgga 1920
ctgagccctg actactgggg cctgggctgc ctcatctatg agatgattga aggccagtca 1980
ccatttcgag gtcgcaagga gaaggttaag cgggaagagg tggatcgccg ggtgctggag 2040
actgaggaag tgtattcctc caagttctct gaagaggcca agtccatctg caacatgctg 2100
ctcaccaaag actcgaagca gaggctgggc tgccaggagg agggggccgc cgaggtcaag 2160
aggcaccctt tcttcaggaa catgaacttt aagcgcctgg aggctgggat gttggaccct 2220
cccttcgttc cagatccccg ggctgtatac tgcaaggatg tgctggacat tgagcagttc 2280
tccactgtga aaggtgtcaa cctggaccat acggacgatg atttttactc aaagttctct 2340
acaggctctg tgccaattcc atggcaaaat gagatgatag aaacagaatg tttcaaggag 2400
ctgaatgtgt tcggacctaa cggtaccctc tcaccagacc tgaacagaag tcagcctcca 2460
gaaccgccaa agaaagggct gttccacaga ctcttcaggc gtcagcatca aagcaattcc 2520
aagagttcac ctactcctaa gaccagttgt aaccaccgaa taaattcaaa ccacatcaat 2580
tcaaactcca ctggaagcag ctagtttcgg ctctggcctt gaagtcaaaa gtggaaccag 2640

ctcagagcct tctacttgga agcagaactt gtagccaggg gagcttccac tgtggctcag 2700

tggccagcaa agctccagtg ggaactaaga taggagaccg ttcccccaat aacaaacctc 2760

caagtttctc aaagaaattt ccactcaggt ctgttttcca aggtggcccc aagctggggt 2820

ggactagaat tttccttgtt gaacattgca atagaaaccc aatgggatat gacagcttgc 2880

acggatttta atagcatcct aactagaact gaattttgtc tttattattt ttaaaggaaa 2940

gttttgtaaa tttctctatt gtctctgttt acattttgta tatttgtatt taagtgaaag 3000

tcagactttg agggtgtata ttttctgtgc agccactgtt aagccatgtg ttttaagaca 3060

ttttagattg gagggggggt tacaaaaatg tgactctaga cttccagagc ctcaaaagag 3120

ataatgtttt tattaaatat agaaaatatc tcacttttta cctttaaaaa aaaaaaaaaa 3180

aa 3182

<210> 11
<211> 2287
<212> DNA
<213> Mus musculus

<400> 11
gcccccttgg ctgtgactct ctcgctcggt gtcctagagg tccttagtgt ggactggcca 60

cttgggtggc ccaaccttgc gtgccctgga gtaggcagag gtgaagaggg gctacgagtg 120

gctccgccgg cagccttcag acatcggtga ccttgtgggc tcccacgcag agggtctgga 180

gacatggcca gaaaggctct caagcttgct tcatggacca gcgtggctct tgctgcctcc 240

ggtgtctacc tctacagtaa caactacttg gaccctaatg actttggcgc tgtcagggtg 300

ggcagagctg ttgctacgac agctgtcatc agctatgact acctcacctc cctgaggagt 360

gtcccatatg gctctgagga gtatttgcag cgtcgatccc aggtgcacct ccgctctgcc 420

aggcgtctct ttgagctctg ctgtgccaac cggggcactt tcatcaaggt gggccagcac 480

ctgggggcgc tggactacct gctgccagaa gagtacacca gcacactgaa ggtgttgcac 540

agtcaagccc cacagagcag catgcaagag gtccggcagg tcatccgaga agacctgggc 600

aaggagatcc acgatttgtt cctgagcttc gatgacaccc ctcttggggc agcctccctg 660

gcccaggtcc acaaggcggt gttgcatgat ggtcggacag tggcagttaa ggtccagcac 720

ccgaaggttc aggctcaaag ctctaaggac attctcctga tggaggtgct tgtcctggcc 780

gtgaagcaac ttttcccaga ttttgaattc atgtggctgg tggatgaagc gaagaagaac 840

ctgcctctcg agttggactt cctgaatgaa gggaggaacg ctgagaaagt ggcccacatg 900

ctcaggcact ttgacttcct aaaggttccc cagatccact gggagctgtc taccaagagg 960

gtgctcctga tggaatttgt agagggaggt caagtcaacg acagggccta catggagaag 1020

aaccagatcg atgtgaatga gatctcctgc cacctgggca agatgtacag tgagatgatc 1080

tttgtcaatg gcttcgtgca ctgtgacccc cacccaggca acgtactggt acggaagcgt 1140

cctgacacgg gcaaggctga gattgtcctc ttagaccatg ggctttacca ggtgctcacg 1200

gaggagttcc gcctggacta ctgccatctg tggcagtctc tgatctggac tgacatggac 1260

gggctgaaac agtacagcca gcgcctggga gctgcagacc tctacccact gtttgcctgt 1320

atgctgacag cccggtcctg ggactcagtc aaacagggca ttgggcaagc tccagtctct 1380

gctactgagg actcagagat tcgcaataat gcagcctgct acctgcctga gatcagccag 1440

ctccttaacc atgtgcctcg ccagatgctg ctcatcctga agactaatga tctactccgt 1500

agcattgaga ccaccctggg cacgcgctcc agtgccagtt ccttcctcaa catgtctcgg 1560

tgttgtatca gggcgctggc tgaacacaag aagagggatg ccggctcttt cttcagaagg 1620

actcagatat ctttcagtga ggcctttagc ctgtggcaga tcaaccttca tgaacttctg 1680

cttcgagtga gggccttgag gctagcttgc tgggtctcag ctctcctggg ctggctgact 1740

cgggctccac acagaatgtg atggtctttc cctgcccctt cgtagtgtct ttccacacct 1800

cattccttcc ttcacgctgg gacgacccac tgacccatgg ctgcctaggg ttggctgtgg 1860

tcccagagtg gtcatccatg gcaccctcat gctctgccat tggagcccct gccttggggt 1920

ggccttgtct tgaggtcctg gaatgtcctg gagagtgaga tgggataaag caacctctct 1980

cccatctcct gtgtgtgcca ttgacttggt catccctact tttatgagga ctgtgagaat 2040

ggaccaacca cctgtgtcac aggggcgtgt ttaacttgta gggaataagt agaaactcag 2100

aacctgcaga gaacagactt tatatttttg ctgtatcact cccaaagttg tctcgcctca 2160

gtgaggaaga ctgcatctga gagggaacga tgcaactgtg ggctcatgct gtcatggtga 2220

caccttcagt gttatatgta ttgtatatat tgtttattgt aataaaccaa taaacagttt 2280

caaggtt 2287

<210> 12
<211> 4208
<212> DNA
<213> Mus musculus

<400> 12
gagggggctt ccgcttccgg cagcggaggc tgcagcctcg ctctggtccc tgcggctggc 60

ggccgagccg tgtgtctcct cctccgccgc cgccatattg tctgtgtgaa gctaggggag 120

cgcggctatt gccgctgccg cttccaccgc agtgtgaaga aaaagggtct gaaacaaagt 180

cttaccaacg tctgcttttg aacacagtga ctgctggatc tttaaacatc aagttcagct 240

ttgtctgtca acctgtctga catgtcggga cccgtgccaa gcagggccag agtttacaca 300

gatgttaaca cacacagacc ccgagagtac tgggattatg aatcacatgt ggtggaatgg 360

gggaatcaag atgactatca gcttgttcga aaattaggta ggggcaaata cagtgaagtg 420

tttgaagcca tcaacatcac aaataatgaa aaagttgttg ttaaaattct caagccagta 480

aaaaagaaga aaattaagcg tgaaataaag attttggaga atttgagagg tgggcccaac 540

atcatcacac ttgcagacat tgtaaaagac cctgtgtcac gaacccctgc cttggttttt 600

gaacatgtaa acaacacaga cttcaagcaa ttgtaccaga cgttaacaga ctatgacatt 660

cgattttaca tgtatgaaat tctgaaagcc ctggattatt gtcacagcat ggggattatg 720

cacagagatg tgaaacccca taatgtcatg attgatcatg agcacagaaa gcttcggcta 780
atagactggg gtttagctga gttttaccat ccaggccaag agtataatgt ccgagttgct 840
tctcgatatt tcaaaggtcc agagctactt gtagattatc agatgtacga ttatagtttg 900
gatatgtgga gcttgggttg tatgctggcc agtatgatct tccggaagga gccatttttc 960
catggacatg acaattatga tcagttggtg aggatagcca aggttctggg aacagaagat 1020
ttatatgact atattgacaa gtacaacatt gaattagatc cacgtttcaa cgatatcttg 1080
ggcagacact cccgtaagcg atgggaacgc tttgtccaca gtgaaaacca gcatcttgtc 1140
agccctgagg ccttggattt tctggacaag ctgcttcgat atgaccacca gtcacggctc 1200
actgcaagag aggccatgga gcacccttac ttctacactg ttgtgaagga ccaggctcga 1260
atgagttcca ctagcatggc agggggcagc acacctgtca gcagcgccaa tatgatgtca 1320
gggatttctt cagtgccaac tccttcaccc cttggacctc tggcaggctc accagtgatt 1380
gccgctgcca actcacttgg gatacctgtt ccagctgctg ctggcgctca gcagtaatga 1440
cccccatcta ccttctgatg cctgagcaga ggtggggaag tccaccctct cctcgatgca 1500
gcttgcgcct gtttgggagg ggtgagaaca cttcagaagc accgtgtctg aaccgttgct 1560
tgtggattta gtagttgagt cataaaaaaa aattataggc tgattttctt tctttctttc 1620
tttctttttt tttttttttt tttttttttt taaaacgtgg acttttcata actcagggga 1680
ttccctgaaa aaattacctg caggtggaat atttcatgga caaaattttt tctccccttc 1740
caaattcagt ttatcaccac taaagaacaa agataaactg gcctcagtcc cagctgctgc 1800
gtttgggtgg aaatttcatc cccttcccac gttgctcttt ccacattgta cactcttgtg 1860
gcttatatct catgttcttc tccagagatt gtaaactgta gctcatcagg ggaggggggg 1920
ggagggggga ggacccagtc catcctacaa cactgttctg ctggctggcc acttcatctc 1980
ttctctccag gactgcagcc ctggcttact ccggctctgg tggaggtggg tctgtcttat 2040
ttacatcaag atgttaaaag tctacccaat gtgcagacag atccaaagct tttttttctg 2100
aacgaatcat gtcttactaa cctaacccta aatgatttta attttagttg agtttctatt 2160
tgtgatactt tccctttctg ggctcctact ttggtctttc ccttttcgtc tccagacatg 2220
ctatgcttca tagctgctag gagagggaag gcaaaatctt tttcagtttt ctttgacttg 2280
gccattttga agacagttag ttagtgggca taatttattg ctttttaaaa aagaatattt 2340
gtccatataa gtttcatgct agcactctta tgagctaatg ggtgatgtgg ccgcactggg 2400
ttcattttaa gctttgtcct ttctcccccc tgccttgccc tttcttcaca tgccatccag 2460
tgaggagccc tgctctcaga attggaagtg tgttttttga gaggcaggag cagagccact 2520
atctccagtg aaccagagat ggtagacctg taattgtggg aaactatcag ttctcttgtt 2580
atggccctgc cgttccttgc tgtgtgtata tatataatac tttgtccttc atatgtgaaa 2640
gatccagtgt tggaattctt tggtgtaaat aaacgtttgg ttttatttat caaggttaga 2700
tttaagttcc ctgtgtaaag gtctcgctgg gtgggtgtct cattcaaacc tgaggggact 2760

acagcccaga ccatggaagc ttcccaaggc cctcattttt atacaaccct catttgaccc 2820

atggtactgg gcagggcaga gaggccttaa aaaaagcacc acaagccaaa gcgtctctgg 2880

ggattaagga tcctttgcca taaaactcat tctgtgtctc agcattgcag ggattgggga 2940

taggaaaggc gccagttttt gtgtgtttgt gtgtgtataa agtggacttt ccactgtaat 3000

ccagccacct aagtttatca ggtgcttcac tgaggaagcc tagtttttta agcacaatag 3060

caaaacatca gctctgtatt ttctcctgtt atttcattac agtagctgcc tgtggggact 3120

aggaaaacaa atcttccaac atattttaag gcctaaaatc ttagttcccc attctcctac 3180

ctttttagat tcataagcat ttctcacctg gcatcataga taaaacataa ttgtttgggg 3240

agttgaattt aattaactta cctaactttg taacccatct tggctttagt aactttatca 3300

aggtggtggc tttagcgaat gtaatgataa aagtagagga tgctaatgct tttcttaaca 3360

tttctcaacc atagagagag ctaaaaggac tgcagagtga gtttcttctt tttggccact 3420

tgcagtgact atttattgta ctcgattctt gtgtgcccta gcgtgctatg agggtcacca 3480

tgttggattt gtgcagagct gaaagcacca ggtgtgccag agatgcagtt tgtgattatg 3540

tttgcactgg atcgtgattt gaacaagaca cttagactga tgaattcttt cttttgaggt 3600

ggaaaggaaa gttgtagatc aggactctac accctgctca cagctcagaa gtgtaggtgt 3660

gtctgaggct ggtgtggacc gctgtggata actgggcctc ctcacacatc aaacctgtgc 3720

aagagctgag ggtaatgcag aggactctgg tgtgccaccg aagagccctt taaataaatc 3780

gaagcttaaa agaaaaaaag gagtcattgc agaaatgtgg agagttttaa aagatctgag 3840

tgggtggggg gtagggaggg tcagggttta cagagtaaca acttctgtgt tgtaaattac 3900

ctcatctgta caccttgtat tgggacacta tttctcagca cttcctgtgt ctgtattgct 3960

tagacggcgg gatggatgcc aatatgaaac agctgtgttg gcagttactc tgaaagtatt 4020

ttttaacttt gttttgaagt gtgcccttcc cccctttaag tttaaagtca gaattcccct 4080

ccttgattta ttggctgtaa ttgtattgta tttggtatga ctaagacata actgtgctgg 4140

gaagaagttg tgccatgaag gtctttaatt ttttttttta aataaaaaca tttaaatcaa 4200

tgaaaaaa 4208

<210> 13
<211> 806
<212> PRT
<213> Mus musculus

<400> 13

Met Trp Phe Phe Ala Arg Asp Pro Val Arg Asp Phe Pro Phe Glu Leu
1               5                   10                  15

Ser Pro Glu Pro Pro Glu Gly Gly Pro Pro Gly Pro Trp Ile Leu His
            20                  25                  30

Arg Gly Arg Lys Lys Ala Thr Gly Ser Ala Val Ser Ile Phe Val Tyr

35                  40                  45

Asp Val Lys Pro Gly Ala Glu Glu Gln Thr Gln Val Ala Lys Ala Ala
    50                  55                  60

Phe Lys Arg Leu Lys Thr Leu Arg His Pro Asn Ile Leu Ala Tyr Ile
65                  70                  75                  80

Asp Gly Leu Glu Thr Glu Lys Cys Leu His Ile Val Thr Glu Ala Val
                85                  90                  95

Thr Pro Leu Gly Thr Tyr Leu Lys Ala Arg Ala Glu Ala Gly Gly Leu
            100                 105                 110

Lys Glu Gln Glu Leu Ser Trp Gly Leu His Gln Ile Val Lys Ala Leu
        115                 120                 125

Ser Phe Leu Val Asn Asp Cys Asn Leu Ile His Asn Asn Val Cys Met
    130                 135                 140

Ala Ala Val Phe Val Asp Arg Ala Gly Glu Trp Lys Leu Gly Gly Leu
145                 150                 155                 160

Asp Tyr Met Tyr Ser Ala Gln Gly Asn Gly Gly Gly Pro Pro Ser Lys
                165                 170                 175

Gly Ile Pro Glu Leu Glu Gln Tyr Asp Pro Pro Glu Leu Ala Asp Ser
            180                 185                 190

Ser Ser Arg Ala Val Arg Glu Lys Trp Ser Ala Asp Met Trp Arg Leu
        195                 200                 205

Gly Cys Leu Ile Trp Glu Val Phe Asn Gly Ser Leu Pro Arg Ala Ala
    210                 215                 220

Ala Leu Arg Asn Pro Gly Lys Ile Pro Lys Ser Leu Val Thr His Tyr
225                 230                 235                 240

Cys Glu Leu Val Gly Ala Asn Pro Lys Val Arg Pro Asn Pro Ala Arg
                245                 250                 255

Phe Leu Gln Asn Cys Arg Ala Pro Gly Gly Phe Met Ser Asn Arg Phe
            260                 265                 270

Val Glu Thr Asn Leu Phe Leu Glu Glu Ile Gln Ile Lys Glu Pro Ala
        275                 280                 285

Glu Lys Gln Lys Phe Phe Gln Glu Leu Ser Lys Ser Leu Asp Ser Phe
    290                 295                 300

Pro Glu Asp Phe Cys Arg His Lys Val Leu Pro Gln Leu Leu Thr Ala

305                 310                 315                 320

Phe Glu Phe Gly Asn Ala Gly Ala Val Val Leu Thr Pro Leu Phe Lys
                325                 330                 335

Val Gly Lys Ser Leu Arg Ala Glu Glu Tyr Gln Glu Lys Ile Ile Pro
            340                 345                 350

Val Val Val Lys Met Phe Ser Ser Thr Asp Arg Ala Met Arg Ile Arg
        355                 360                 365

Leu Leu Gln Gln Met Glu Gln Phe Ile Gln Tyr Leu Asp Glu Pro Thr
    370                 375                 380

Val Asn Thr Gln Ile Phe Pro His Val Thr His Gly Phe Leu Asp Thr
385                 390                 395                 400

Asn Pro Ala Ile Arg Glu Gln Thr Val Lys Ser Met Leu Leu Leu Ala
                405                 410                 415

Pro Lys Leu Asn Glu Ala Asn Leu Asn Val Glu Leu Met Lys His Phe
            420                 425                 430

Ala Arg Leu Gln Ala Lys Asp Asp Gln Gly Pro Ile Arg Cys Asn Thr
        435                 440                 445

Thr Val Cys Leu Gly Lys Ile Gly Ser Tyr Leu Ser Ala Ser Thr Arg
    450                 455                 460

His Arg Val Leu Thr Ser Ala Phe Ser Arg Ala Thr Lys Asp Pro Phe
465                 470                 475                 480

Ala Pro Ser Arg Val Ala Gly Val Leu Gly Phe Ala Ala Thr His Asn
                485                 490                 495

Leu Tyr Ser Met Asp Asp Cys Ala His Lys Ile Leu Pro Val Leu Cys
            500                 505                 510

Gly Leu Thr Val Asp Pro Glu Lys Ser Val Arg Asp Gln Ala Phe Lys
        515                 520                 525

Thr Ile Arg Ser Phe Leu Ser Lys Leu Glu Ser Val Ser Glu Asp Pro
    530                 535                 540

Thr Gln Leu Ala Glu Val Glu Lys Asp Val His Ala Ala Ser Ser Pro
545                 550                 555                 560

Gly Thr Gly Gly Ala Ala Ala Ser Trp Ala Gly Trp Ala Val Thr Gly
                565                 570                 575

Val Ser Ser Leu Thr Ser Lys Leu Ile Arg Ala His Pro Thr Pro Val

580                 585                 590

Pro Ser Asp Thr Thr Val Pro Gln Arg Pro Val Pro Glu Gly Asn Pro
        595                 600                 605

Ala Pro Ala Pro Ala Leu Ala Gln Ala Ile Pro Ala Thr Ser Gly His
    610                 615                 620

Trp Glu Thr Gln Glu Asp Lys Asp Thr Ala Glu Asp Ser Ala Thr Ala
625                 630                 635                 640

Asp Arg Trp Asp Asp Glu Asp Trp Gly Ser Leu Glu Gln Glu Ala Glu
                645                 650                 655

Ser Val Leu Ala Gln Gln Asp Asp Trp Ser Ala Lys Gly Gln Gly Ser
            660                 665                 670

Arg Ala Gly Gln Ile Asn His Pro Asp His Lys Ser Leu Glu Ser His
        675                 680                 685

Trp Ser Ser Trp Glu Val Glu Gly Ser Trp Asp Gln Gly Trp Gln Glu
    690                 695                 700

Pro Ser Ser Val Glu Pro Pro Pro Glu Gly Thr Arg Leu Ala Ser Glu
705                 710                 715                 720

Tyr Asn Trp Gly Gly Ala Glu Pro Ser Asp Lys Gly Asp Pro Phe Ala
                725                 730                 735

Ala Leu Ser Val Arg Pro Ser Ala Gln Pro Arg Pro Asp Pro Asp Ser
            740                 745                 750

Trp Gly Glu Asp Asn Trp Glu Gly Leu Glu Ala Glu Ser Arg Gln Val
        755                 760                 765

Lys Ala Glu Leu Ala Arg Lys Lys Arg Glu Glu Arg Arg Arg Glu Met
    770                 775                 780

Glu Ala Lys Arg Ala Glu Lys Lys Thr Thr Lys Gly Pro Met Lys Leu
785                 790                 795                 800

Gly Ala Arg Lys Leu Asp
                805

<210>  14
<211>  590
<212>  PRT
<213>  Mus musculus

<400>  14

Met Glu Leu Glu Asn Ile Val Ala Asn Thr Val Leu Leu Lys Ala Arg
1               5                   10                  15

Glu Gly Gly Gly Gly Lys Arg Lys Gly Lys Ser Lys Lys Trp Lys Glu
            20                  25                  30

Ile Leu Lys Phe Pro His Ile Ser Gln Cys Glu Asp Leu Arg Arg Thr
        35                  40                  45

Ile Asp Arg Asp Tyr Tyr Ser Leu Cys Asp Lys Gln Pro Ile Gly Arg
    50                  55                  60

Leu Leu Phe Arg Gln Phe Cys Glu Thr Arg Pro Gly Leu Glu Cys Tyr
65                  70                  75                  80

Ile Gln Phe Leu Asp Leu Val Ala Glu Tyr Glu Ile Thr Pro Asp Glu
                85                  90                  95

Asn Leu Gly Ala Lys Gly Lys Glu Ile Met Thr Lys Tyr Leu Thr Pro
            100                 105                 110

Lys Ser Pro Val Phe Ile Ala Gln Val Gly Gln Asp Leu Val Ser Gln
        115                 120                 125

Thr Glu Lys Lys Leu Leu Gln Ser Pro Cys Lys Glu Leu Phe Ser Ala
    130                 135                 140

Cys Ala Gln Ser Val His Asp Tyr Leu Lys Gly Asp Pro Phe His Glu
145                 150                 155                 160

Tyr Leu Asp Ser Met Tyr Phe Asp Arg Phe Leu Gln Trp Lys Trp Leu
                165                 170                 175

Glu Arg Gln Pro Val Thr Lys Asn Thr Phe Arg Gln Tyr Arg Val Leu
            180                 185                 190

Gly Lys Gly Gly Phe Gly Glu Val Cys Ala Cys Gln Val Arg Ala Thr
        195                 200                 205

Gly Lys Met Tyr Ala Cys Lys Arg Leu Glu Lys Lys Arg Ile Lys Lys
    210                 215                 220

Arg Lys Gly Glu Ser Met Ala Leu Asn Glu Lys Gln Ile Leu Glu Lys
225                 230                 235                 240

Val Asn Ser Gln Phe Val Val Asn Leu Ala Tyr Ala Tyr Glu Thr Lys
                245                 250                 255

Asp Ala Leu Cys Leu Val Leu Thr Ile Met Asn Gly Gly Asp Leu Lys
            260                 265                 270

Phe His Ile Tyr Asn Met Gly Asn Pro Gly Phe Glu Glu Glu Arg Ala
        275                 280                 285

Leu Phe Tyr Ala Ala Glu Ile Leu Cys Gly Leu Glu Asp Leu His Arg
    290                 295                 300

Glu Asn Thr Val Tyr Arg Asp Leu Lys Pro Glu Asn Ile Leu Leu Asp
305                 310                 315                 320

Asp Tyr Gly His Ile Arg Ile Ser Asp Leu Gly Leu Ala Val Lys Ile
                325                 330                 335

Pro Glu Gly Asp Leu Ile Arg Gly Arg Val Gly Thr Val Gly Tyr Met
            340                 345                 350

Ala Pro Glu Val Leu Asn Asn Gln Arg Tyr Gly Leu Ser Pro Asp Tyr
        355                 360                 365

Trp Gly Leu Gly Cys Leu Ile Tyr Glu Met Ile Glu Gly Gln Ser Pro
    370                 375                 380

Phe Arg Gly Arg Lys Glu Lys Val Lys Arg Glu Glu Val Asp Arg Arg
385                 390                 395                 400

Val Leu Glu Thr Glu Glu Val Tyr Ser Ser Lys Phe Ser Glu Glu Ala
                405                 410                 415

Lys Ser Ile Cys Asn Met Leu Leu Thr Lys Asp Ser Lys Gln Arg Leu
            420                 425                 430

Gly Cys Gln Glu Glu Gly Ala Ala Glu Val Lys Arg His Pro Phe Phe
        435                 440                 445

Arg Asn Met Asn Phe Lys Arg Leu Glu Ala Gly Met Leu Asp Pro Pro
    450                 455                 460

Phe Val Pro Asp Pro Arg Ala Val Tyr Cys Lys Asp Val Leu Asp Ile
465                 470                 475                 480

Glu Gln Phe Ser Thr Val Lys Gly Val Asn Leu Asp His Thr Asp Asp
                485                 490                 495

Asp Phe Tyr Ser Lys Phe Ser Thr Gly Ser Val Pro Ile Pro Trp Gln
            500                 505                 510

Asn Glu Met Ile Glu Thr Glu Cys Phe Lys Glu Leu Asn Val Phe Gly
        515                 520                 525

Pro Asn Gly Thr Leu Ser Pro Asp Leu Asn Arg Ser Gln Pro Pro Glu
    530                 535                 540

Pro Pro Lys Lys Gly Leu Phe His Arg Leu Phe Arg Arg Gln His Gln
545                 550                 555                 560

Ser Asn Ser Lys Ser Ser Pro Thr Pro Lys Thr Ser Cys Asn His Arg
                565                 570                 575

Ile Asn Ser Asn His Ile Asn Ser Asn Ser Thr Gly Ser Ser
            580                 585                 590

<210>  15
<211>  551
<212>  PRT
<213>  Mus musculus

<400>  15

Met Ala Arg Lys Ala Leu Lys Leu Ala Ser Trp Thr Ser Val Ala Leu
1               5                   10                  15

Ala Ala Ser Gly Val Tyr Leu Tyr Ser Asn Asn Tyr Leu Asp Pro Asn
            20                  25                  30

Asp Phe Gly Ala Val Arg Val Gly Arg Ala Val Ala Thr Thr Ala Val
        35                  40                  45

Ile Ser Tyr Asp Tyr Leu Thr Ser Leu Arg Ser Val Pro Tyr Gly Ser
    50                  55                  60

Glu Glu Tyr Leu Gln Arg Arg Ser Gln Val His Leu Arg Ser Ala Arg
65                  70                  75                  80

Arg Leu Phe Glu Leu Cys Cys Ala Asn Arg Gly Thr Phe Ile Lys Val
                85                  90                  95

Gly Gln His Leu Gly Ala Leu Asp Tyr Leu Leu Pro Glu Glu Tyr Thr
            100                 105                 110

Ser Thr Leu Lys Val Leu His Ser Gln Ala Pro Gln Ser Ser Met Gln
        115                 120                 125

Glu Val Arg Gln Val Ile Arg Glu Asp Leu Gly Lys Glu Ile His Asp
    130                 135                 140

Leu Phe Leu Ser Phe Asp Asp Thr Pro Leu Gly Ala Ala Ser Leu Ala
145                 150                 155                 160

Gln Val His Lys Ala Val Leu His Asp Gly Arg Thr Val Ala Val Lys
                165                 170                 175

Val Gln His Pro Lys Val Gln Ala Gln Ser Ser Lys Asp Ile Leu Leu
            180                 185                 190

Met Glu Val Leu Val Leu Ala Val Lys Gln Leu Phe Pro Asp Phe Glu
        195                 200                 205

Phe Met Trp Leu Val Asp Glu Ala Lys Lys Asn Leu Pro Leu Glu Leu
    210                 215                 220

Asp Phe Leu Asn Glu Gly Arg Asn Ala Glu Lys Val Ala His Met Leu
225                 230                 235                 240

Arg His Phe Asp Phe Leu Lys Val Pro Gln Ile His Trp Glu Leu Ser
                245                 250                 255

Thr Lys Arg Val Leu Leu Met Glu Phe Val Glu Gly Gly Gln Val Asn
            260                 265                 270

Asp Arg Ala Tyr Met Glu Lys Asn Gln Ile Asp Val Asn Glu Ile Ser
        275                 280                 285

Cys His Leu Gly Lys Met Tyr Ser Glu Met Ile Phe Val Asn Gly Phe
    290                 295                 300

Val His Cys Asp Pro His Pro Gly Asn Val Leu Val Arg Lys Arg Pro
305                 310                 315                 320

Asp Thr Gly Lys Ala Glu Ile Val Leu Leu Asp His Gly Leu Tyr Gln
                325                 330                 335

Val Leu Thr Glu Glu Phe Arg Leu Asp Tyr Cys His Leu Trp Gln Ser
            340                 345                 350

Leu Ile Trp Thr Asp Met Asp Gly Leu Lys Gln Tyr Ser Gln Arg Leu
        355                 360                 365

Gly Ala Ala Asp Leu Tyr Pro Leu Phe Ala Cys Met Leu Thr Ala Arg
    370                 375                 380

Ser Trp Asp Ser Val Lys Gln Gly Ile Gly Gln Ala Pro Val Ser Ala
385                 390                 395                 400

Thr Glu Asp Ser Glu Ile Arg Asn Asn Ala Ala Cys Tyr Leu Pro Glu
                405                 410                 415

Ile Ser Gln Leu Leu Asn His Val Pro Arg Gln Met Leu Leu Ile Leu
            420                 425                 430

Lys Thr Asn Asp Leu Leu Arg Ser Ile Glu Thr Thr Leu Gly Thr Arg
        435                 440                 445

Ser Ser Ala Ser Ser Phe Leu Asn Met Ser Arg Cys Cys Ile Arg Ala
    450                 455                 460

Leu Ala Glu His Lys Lys Arg Asp Ala Gly Ser Phe Phe Arg Arg Thr
465                 470                 475                 480

Gln Ile Ser Phe Ser Glu Ala Phe Ser Leu Trp Gln Ile Asn Leu His
                485                 490                 495

Glu Leu Leu Leu Arg Val Arg Ala Leu Arg Leu Ala Cys Trp Val Ser
            500                 505                 510

Ala Leu Leu Gly Trp Leu Thr Arg Ala Pro His Arg Met Arg Arg Glu
        515                 520                 525

Met Glu Ala Lys Arg Ala Glu Lys Lys Thr Thr Lys Gly Pro Met Lys
    530                 535                 540

Leu Gly Ala Arg Lys Leu Asp
545                 550

<210> 16
<211> 391
<212> PRT
<213> Mus musculus

<400> 16

Met Ser Gly Pro Val Pro Ser Arg Ala Arg Val Tyr Thr Asp Val Asn
1               5                   10                  15

Thr His Arg Pro Arg Glu Tyr Trp Asp Tyr Glu Ser His Val Val Glu
            20                  25                  30

Trp Gly Asn Gln Asp Asp Tyr Gln Leu Val Arg Lys Leu Gly Arg Gly
        35                  40                  45

Lys Tyr Ser Glu Val Phe Glu Ala Ile Asn Ile Thr Asn Asn Glu Lys
    50                  55                  60

Val Val Val Lys Ile Leu Lys Pro Val Lys Lys Lys Lys Ile Lys Arg
65                  70                  75                  80

Glu Ile Lys Ile Leu Glu Asn Leu Arg Gly Gly Pro Asn Ile Ile Thr
                85                  90                  95

Leu Ala Asp Ile Val Lys Asp Pro Val Ser Arg Thr Pro Ala Leu Val
            100                 105                 110

Phe Glu His Val Asn Asn Thr Asp Phe Lys Gln Leu Tyr Gln Thr Leu
        115                 120                 125

Thr Asp Tyr Asp Ile Arg Phe Tyr Met Tyr Glu Ile Leu Lys Ala Leu
    130                 135                 140

Asp Tyr Cys His Ser Met Gly Ile Met His Arg Asp Val Lys Pro His
145                 150                 155                 160

Asn Val Met Ile Asp His Glu His Arg Lys Leu Arg Leu Ile Asp Trp
                165                 170                 175

Gly Leu Ala Glu Phe Tyr His Pro Gly Gln Glu Tyr Asn Val Arg Val
            180                 185                 190

Ala Ser Arg Tyr Phe Lys Gly Pro Glu Leu Leu Val Asp Tyr Gln Met
        195                 200                 205

Tyr Asp Tyr Ser Leu Asp Met Trp Ser Leu Gly Cys Met Leu Ala Ser
    210                 215                 220

Met Ile Phe Arg Lys Glu Pro Phe Phe His Gly His Asp Asn Tyr Asp
225                 230                 235                 240

Gln Leu Val Arg Ile Ala Lys Val Leu Gly Thr Glu Asp Leu Tyr Asp
                245                 250                 255

Tyr Ile Asp Lys Tyr Asn Ile Glu Leu Asp Pro Arg Phe Asn Asp Ile
            260                 265                 270

Leu Gly Arg His Ser Arg Lys Arg Trp Glu Arg Phe Val His Ser Glu
        275                 280                 285

Asn Gln His Leu Val Ser Pro Glu Ala Leu Asp Phe Leu Asp Lys Leu
    290                 295                 300

Leu Arg Tyr Asp His Gln Ser Arg Leu Thr Ala Arg Glu Ala Met Glu
305                 310                 315                 320

His Pro Tyr Phe Tyr Thr Val Val Lys Asp Gln Ala Arg Met Ser Ser
                325                 330                 335

Thr Ser Met Ala Gly Gly Ser Thr Pro Val Ser Ser Ala Asn Met Met
            340                 345                 350

Ser Gly Ile Ser Ser Val Pro Thr Pro Ser Pro Leu Gly Pro Leu Ala
        355                 360                 365

Gly Ser Pro Val Ile Ala Ala Ala Asn Ser Leu Gly Ile Pro Val Pro
    370                 375                 380

Ala Ala Ala Gly Ala Gln Gln
385                 390

## Description

The present invention is related to compound capable of modulating the activity and/or expression of certain protein kinases thereby enhancing the expression and/or release of insulin. The invention is further related to methods of identifying said compounds for the treatment of metabolic diseases. The invention is further related to methods of treatment of metabolic diseases, particularly diabetes mellitus type 2.

Diabetes as a leading cause of death in developed countries is a metabolic condition characterized by high blood sugar levels. There are two main types of diabetes: type 1, resulting from insufficient insulin production of the pancreas beta cells, which requires the person to inject insulin; and type 2, resulting from insensitivity of peripheral tissues (such skeletal muscle, liver or adipose tissue) insulin release alterations, and relative insulin deficiency. Diabetes mellitus type 2 is often acquired and accompanied by obesity; it can be treated in first hand by reducing weight, diet and exercise. Type 1 diabetes is a genetic or autoimmune disease; the only effective therapy to date is the supply of exogenous insulin. This therapy does not cure diabetes; the person needs continuous supply of insulin.

The decreased insulin sensitivity of peripheral tissues in type 2 diabetes which accounts for 90% of all cases of the disease is initially compensated by an increased release of insulin by the beta cells of the pancreas. At a certain stage of the disease, the pancreas cannot maintain the increases release of insulin anymore. As disease progresses, drugs which are currently available and elevate insulin release have lead to beta-cell damage and loss of insulin production.

A number of diseases, including cancer, diabetes and inflammation are linked to perturbation of protein kinase mediated cell signaling pathways. For some time, a new class of multiple kinase drugs has been undergoing clinical trials. Some have been approved for various applications, mostly for the treatment of cancer. The targets of these multiple kinase inhibitors like Imanitib or Sunitinib interact at all stages of signal transduction: from the receptor tyrosine kinases which initiate intracellular signaling to second-messenger generators and kinases involved in signaling cascades and finally to those kinases which regulate the cell cycle governing cellular fate.

Several publications have shown the effect of kinase-inhibitors like Sunitinib (Sutent^{®}) and Imatinib (Gleevec^{®}) on diabetes during a period of treatment which leads to a remission of diabetes type 1 or 2 in patients. However, only few kinases could be identified that affect the insulin release or sensitivity specifically to develop a more specific treatment strategy.

The objective of the present invention is to provide targets for the treatment of metabolic diseases such as diabetes, compounds which are useful for raising the blood insulin level by enhancing the insulin release of the pancreatic beta cells and methods for identifying such compounds. This goal is achieved by the compounds which bind to regulating protein kinases according to claim 1 as well as by the methods for identifying such compounds and the disclosed targets SCYL1, ADCK1, GRK5 and CSNK2A1. Further advantageous embodiments, aspects and details of the invention are evident from the pending claims, the description, the examples and the figures.

### Short description

The invention refers particularly to a modulator for the inhibition of the activity of protein kinases, wherein kinases selected from the group of consisting of SCYL1, ADCK1, GRK5 and CSNK2A1 are preferred targets for inhibition, and wherein a preferred goal of the inhibition is the treatment of metabolic diseases, more preferred of a disease of the carbohydrate metabolism, more preferred of diabetes, more preferred of diabetes mellitus type 2, and most preferred for the up-regulation of insulin production and/or release of insulin. The invention refers further to a modulator for the inactivation, degradation, downregulation, intercalation of at least one nucleic acid selected from the group consisting of the nucleic acid encoding SCYL1, the nucleic acid encoding ADCK1, the nucleic acid encoding GRK5 and the nucleic acid encoding CSNK2A1 for the treatment of metabolic diseases, more preferred of a disease of the carbohydrate metabolism, more preferred of diabetes, more preferred of diabetes mellitus type 2, and most preferred for the up-regulation of insulin production and/or release of insulin.

The said modulator can be chosen from the group comprising a small molecule, an RNA molecule, a siRNA molecule, a miRNA molecule, or a precursor thereof, an antisense oligonucleotide, an aptamer, a polypeptide, an antibody, a ribozyme, or Sunitinib, wherein RNA, peptides and antibodies are preferred modulators.

The invention refers further to a pharmaceutical composition comprising a modulator for the treatment of metabolic diseases, more preferred of a disease of the carbohydrate metabolism, more preferred of diabetes, more preferred of diabetes mellitus type 2, and most preferred for the up-regulation of insulin production.

The invention refers further to a method for screening for a modulator for treatment of a metabolic disease, wherein the method comprises providing a test compound for contacting at least one polypeptide or nucleic acid coding for at least one polypeptide selected from the group consisting of SCYL1, ADCK1, GRK5 and CSNK2A1 polypeptide, detecting the binding of said test compound to the SCYL1, ADCK1, GRK5 or CSNK2A1 polypeptide or nucleic acid coding for at least one polypeptide, and determining the activity of the SCYL1, ADCK1, GRK5 or CSNK2A1 polypeptide in the presence of said test compound.

For identification of an inventive compound the invention further provides a kit comprising the SCYL1, ADCK1, GRK5 and/or CSNK2A1 polypeptides, the nucleic acid encoding SCYL1, the nucleic acid encoding ADCK1, the nucleic acid encoding GRK5 and the nucleic acid encoding CSNK2A1, a cell line with a glucose dependent insulin production, and a control compound known to affect the insulin production by binding the SCYL1, ADCK1, GRK5 and/or CSNK2A1 polypeptide.

The invention further provides a method for treatment of a metabolic disease comprising administering a subject in need thereof a therapeutically effective amount of at least one modulator for inhibition or activation of at least one of the tyrosine kinases selected from the group consisting of SCYL1, ADCK1, GRK5 and CSNK2A1, or inactivation, degradation, downregulation, intercalation or activation of at least one nucleic acid selected from the group consisting of the nucleic acid encoding SCYL1, the nucleic acid encoding ADCK1, the nucleic acid encoding GRK5 and the nucleic acid encoding CSNK2A1.

### Description

It was surprisingly found that Sunitinib has an effect on insulin release in a dose dependent manner. It was found that this effect was due to the inhibition of certain kinases by Sunitinib. It was further surprisingly found that the inhibition of identified protein kinases by other compounds results in the enhanced release of insulin. It was further found that the combined inhibition of certain protein kinase pairs results in the release of additional insulin. It was further found that the inhibition of certain protein kinase pairs results in an insulin releasing effect which equals the effect after Sunitinib treatment.

Kinases are enzymes which catalyze the transfer of a phosphate group from a donor onto an acceptor. Phosphorylated is a nucleophil functional group, such as hydroxyl-, carboxy-, guanidino-, thiol-, or imidazole groups. Kinases which phosphorylate proteins are called protein kinases. Protein kinases play a particular role in cellular signal transduction. They are usually categorized by their substrate; thus protein kinases may be roughly divided into two groups: protein tyrosine kinases (PTK), which phosphorylate the hydroxyl group of the tyrosine, and serine/threonine kinases (STK), which phosphorylate the hydroxyl groups of the serine or threonine. Examples for PTK are Kinases of the EphA family, Lck, Scyl, HCK, BLK, ITK, TEC, EXK, BTK, CTK, Fyn, Fgr, Src, Yes, Lyn, Tyk, JAK-family, CSK, Arg, Abl, Fes, Fer, Srm, Brk, Syk, ZAP70, FAK, PYK2, DDR, TRK, HER-family, FGFR-family, FLT, Mer, Reg, Axl, Met, Ron, RYK, InsR, IGF1R, LTK, ALK, Ros, Lmr-family. STK are mainly regulated by cAMP, cGMP, DAG, Ca²⁺ or Calmodulin, 1,2-Diacylglycerine, PIP3 and other phospholipid-derivates. Examples for STK include enzymes of the families protein kinase A, B and C, GRK-family, MAST-family, CSNK, PRK, NDR, p70S6K, MSK, MRCK, ROCK, CRIK, DMPK, PKN, Nek, Pim, Aur, SSTK, TSSK, Obscn, skMLCK, DRAK, FAPK, BRSK, MNK, PKD, MAP, PIK3, CHBK, PIP, CERK, TLK, CASK, AKT, KCNH2, GSK, FUK.

Other categorizations are based on the activating compounds of the kinases, or the activating mechanism, or certain catalytic domains or specific amino acid sequences of the kinases. The sum of all kinases in one cell is called kinome.

Based on their function the protein kinases present a very important control mechanism in signal transduction, and are controlling various anabolic and metabolic pathways. On the basis of their importance dysfunctions of protein kinases in cellular pathways are the cause for many diseases, like cancer, metabolic diseases, cardiovascular diseases, arteriosclerosis, thyroid disorders, endocrinological diseases, gastroenterological diseases, inflammation, immune disorders, disorders affecting growth and development, hematological diseases, respiratory diseases, muscle skeleton diseases, neurological diseases, and urological disease. This makes these enzymes attractive molecular targets for therapy.

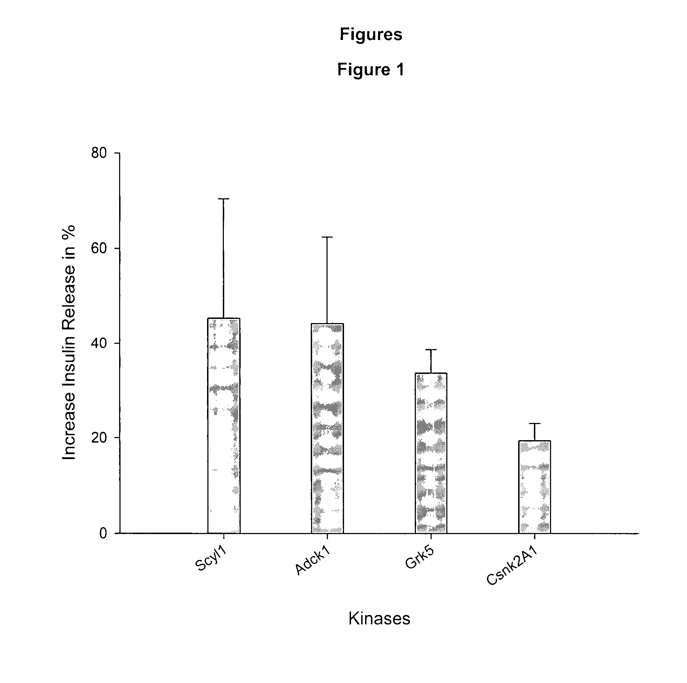

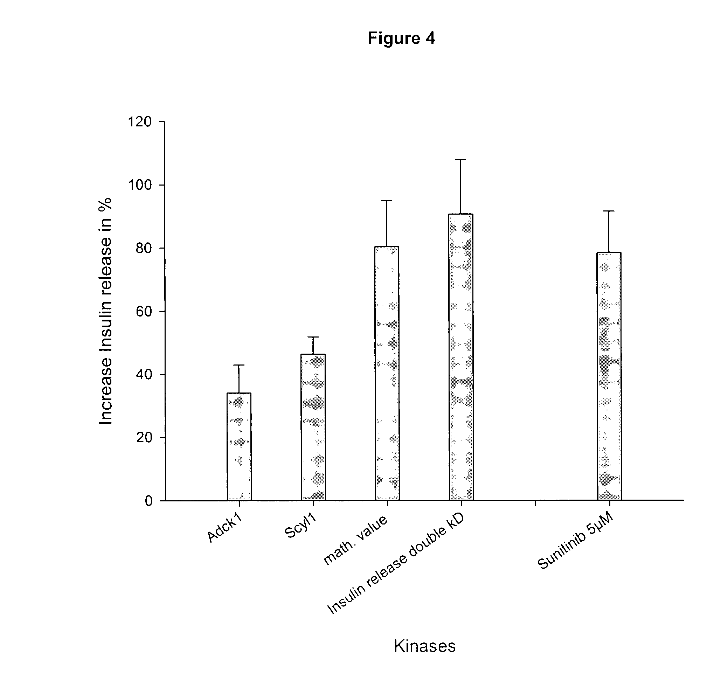

Surprisingly the kinases SCYL1, ADCK1, GRK5 and CSNK2A1 were identified to modulate the insulin release in a significant manner (Fig. 1 and 4). These kinases are preferred targets for the therapy of metabolic disease, preferred of diabetes, more preferred of diabetes type 2, and most preferred to elevate the blood level of insulin. Kinases which present a target for the therapy of metabolic diseases are basically all kinases which affect metabolic pathways. According to the invention, kinases which have been found to affect or modulate the insulin release or sensitivity and are thus preferred targets for a therapy of diabetes, preferably diabetes type 2, are kinases the inhibition of which is correlated to a significant increase of insulin release, namely SCYL1, ADCK1, GRK5 and CSNK2A1.

The present invention refers particularly to a modulator for
a) inhibition of at least one of the protein kinases selected from the group consisting of SCYL1, ADCK1, GRK5 and CSNK2A1, or
b) inactivation, degradation, downregulation, intercalation of at least one nucleic acid selected from the group consisting of the nucleic acid encoding SCYL1, the nucleic acid encoding ADCK1, the nucleic acid encoding GRK5 and the nucleic acid encoding CSNK2A1,
for the treatment of a metabolic disease.

Thus, in a preferred embodiment of the invention, the action of the protein kinases SCYL1, ADCK1, GRK5 and/or CSNK2A1 is blocked by an inhibitor. It is sufficient to block one of the kinases SCYL1, ADCK1, GRK5 or CSNK2A1 and especially one of the kinases SCYL1, ADCK1 or GRK5. However, inhibition of two kinases especially two of SCYL1, ADCK1, GRK5 may further increase the therapeutic effect. Consequently, in a further preferred embodiment of the invention, two of the kinases are inhibited in combination for an increased insulin release. The possible combinations are SCYL1/ADCK1, SCYL1/GRK5, and ADCK1/GRK5. In a further preferred embodiment of the invention, all three enzymes SCYL1, ADCK1, and GRK5 may be inhibited at once for an increased insulin release. In order to achieve the inhibition of two of the kinases a single inhibitor or a combination of two inhibitors could be used. In order to achieve the inhibition of the three kinases SCYL1, ADCK1, and GRK5 a single inhibitor or a combination of two inhibitors or a combination of three inhibitors could be used. Enzyme inhibitors are, in general, molecules which bind to enzymes and decrease their activity. The binding of an inhibitor can stop a substrate from entering the enzymes active site, compete with the substrate for the binding site, or hinder the enzyme from catalyzing its reaction. Inhibitor binding can be reversible or irreversible. Protein kinase inhibitors are a type of enzyme inhibitors which specifically block the action of one or more protein kinases.

In another preferred embodiment, the action of the of the protein kinases SCYL1, ADCK1, GRK5 and/or CSNK2A1 is impeded by interference of their nucleic acid, which can be both DNA and RNA, by inactivation, degradation, downregulation, or intercalation. Inactivation of a nucleic acid can happen for instance by methylation of nucleotides, insertion, deletion, nucleotide exchange, cross linkage, or strand break/damage. Nucleic acids can be degraded down to single nucleotides by temperature, chemicals, enzymes, and particularly RNA by deadenylation or 5'decay or 3'decay. Downregulation of DNA or RNA is referred to as diminished expression of these nucleic acids and can happen by binding of repressors, which are usually polypeptides, but can also happen by chemical or structural changes or modifications of the nucleic acids. Intercalation is the reversible inclusion of a molecule between two other molecules. In nucleic acids, intercalation occurs when ligands of an appropriate size and chemical nature fit themselves in between base pairs.

The term modulator as it appears herein refers to a molecule that is able to change the activity of the SCYL1, ADCK1, GRK5 and/or CSNK2A1 polypeptides. This change may be an increase or a decrease in enzymatic activity, binding characteristics, or functional, immunological or any other biological property of the polypeptides. In order to enhance the insulin release, a decrease of the enzymatic activity is advantageous.

According to the invention, modulators for the inhibition of SCYL1, ADCK1, GRK5 and CSNK2A1 can be molecules like small molecules, RNA or DNA molecules, siRNA or precursor thereof, miRNA or precursors thereof, ribozymes, DNA or RNA antisense oligonucleotides, aptamers, antibodies or fragments thereof, peptides, polypeptides, cyclopeptides, or drugs like sunitinib, imatinib, dasanitib, and sorafenib.

The inventive modulators are also referred to as compounds or test compounds. They modulate the expression and/or activity of the polypeptides of the invention can be identified using one or more assays, alone or in combination. Test compounds used in the screening are not particularly limited. They can be either artificial or natural.

The term small molecule refers to low molecular weight organic compound which is by definition not a polymer. It the field of pharmacology, it is usually restricted to a molecule that also binds with high affinity to a biopolymer such as proteins, nucleic acids, or polysaccharides. Small molecules are broadly used as enzyme inhibitors, thus they are preferred modulators for the inhibition of the preferred kinases in the present invention.

Small interfering RNA (short interfering RNA, silencing RNA, siRNA) is a class of double-stranded RNA-molecules, which are 19-30 nucleotides, preferably 20-25 nucleotides long. siRNAs are involved in the RNA-interference of the expression of a specific gene. siRNAs are cut from long doublestranded RNAs by the RNase III Dicer. They can also be derived by chemical synthesis. They also play a role in antiviral mechanisms or in shaping the chromatin structure of a genome. In molecular research, synthetic siRNAs can also be used in RNA-interference (RNAi) to regulate down the expression of specific target genes. With their ability to knock down essentially any gene of interest, siRNAs have been used to knock down protein kinases to investigate their role in insulin production (Fig. 1 - Fig. 4). siRNAs are preferred modulators for inhibition of the preferred kinases in the present invention.

MicroRNAs (miRNAs) are posttranscriptional regulators that bind to complementary sequences in the 3'UTR of mRNA transcripts, usually resulting in gene silencing. They are short RNA molecules which are about 22 nucleotides long. As miRNAs have been shown to play multiple roles in transcript degradation, sequestering and transcriptional suppression, they are also preferred modulators for inhibition of the preferred kinases in the present invention.

Precursor molecules, e.g. precursor molecules of siRNA and/or miRNA may be a substrate for the siRNA/miRNA-biogenesis-apparatus of the target cell. This comprises, for example, RNA precursor molecules such as double-stranded RNA (dsRNA) or short hairpin RNA-molecules (shRNA), which are processed by endonucleases such as Drosha and/or Pasha to siRNA-molecules or miRNA-molecules, respectively. For this reason, for example dsRNA-molecules or short hairpin RNA-molecules (shRNA) having a length of more than 27 nucleotides, preferably more than 30 up to 100 nucleotides or longer, and mostly preferred dsRNA-molecules having a length of 30-50 nucleotides, can be used.

Further precursor molecules according to the invention may be DNA constructs encoding dsRNA, shRNA, siRNA and/or miRNA, whereby the coding elements are controlled by regulatory elements allowing an expression of dsRNA, shRNA, siRNA and/or miRNA in the target cell. Examples for such control elements are polymerase II promoters or polymerase III promoters such as, for example, U6 or H1.

Ribozymes are catalytic RNAs which possess a well defined structure that enables them to catalyze a chemical reaction. Apart from naturally occurring ribozymes they can be made artificially and be tailored to interact with nucleic acids and proteins. Ribozymes are also preferred modulators for inhibition of the preferred kinases in the present invention.

Antisense oligonucleotides are single strands of DNA or RNA that are complementary to a chosen sequence. They are between 10 and 35 nucleotides long, preferably about 20 - 25 nucleotides. Antisense DNA oligonucleotides can target specific, complementary RNA, and upon binding DNA/RNA hybrids are formed. Antisense RNA oligonucleotides can bind to mRNA by binding to mRNA strands. Antisense oligonucleotides are also preferred modulators for inhibition of the preferred kinases in the present invention.

Aptamers are oligonucleic acid (DNA or RNA aptamers) or peptide molecules (peptide aptamers) that bind to a specific target molecule. Aptamers can be used for therapeutic purposes as macromolecular drugs. Aptamers can be created by selecting them from a large random sequence pool. Aptamers are also preferred modulators for inhibition of the preferred kinases in the present invention.

Antibodies are proteins which bind very specifically to antigens. They are formed by the immune system of the body in response to antigen presence. They can be formed for virtually any structure and are thus valuable tools for direct interaction with certain molecules. Recombinant techniques are used to generate antibodies and antibody fragments which basically consist of the binding moieties of the antibodies. Antibodies are also preferred modulators for inhibition of the preferred kinases in the present invention.

Peptides are stretches of amino acid residues which are connected by peptide bonds. They can be seen as little proteins. Peptides are usually up to 100 amino acids long, from which on the compound is referred to as a protein. Polypeptides are peptides of at least 10 amino acids. Cyclopeptides are formed by two, three or more amino acids, which form ring structures and have thus no C- and N-terminal amino acids. Peptides are preferred, polypeptides more preferred modulators for inhibition of the preferred kinases in the present invention.

The drugs sunitinib, imatinib, dasatinib, and sorafenib are small molecules which inhibit protein kinases which are mainly used in cancer treatment. The drug sunitinib is a preferred modulator for inhibition of the preferred kinases in the present invention.

Metabolic diseases refer to diseases and conditions characterized by pathological disorders of the metabolism. They are mainly characterized by enzyme defects and abnormalities in the regulating system leading to a pathological enrichment of substrates, lack of metabolic products, failure of producing energy, of regeneration of cellular constituents, of elimination of metabolic products, and of maintenance of homeostasis. They can be acquired or be a genetic disease. Metabolic disorders include, but are not limited to, obesity and diabetes (e.g., diabetes type I, diabetes type II, MODY, and gestational diabetes), hypoglycemia, amyloidosis, branched chain disease, hyperaminoacidemia, hyperaminoaciduria, disturbances of the metabolism of urea, hyperammonemia, mucopolysaccharidoses e. g. Maroteaux-Lamy syndrom, glycogen storage diseases and lipid storage diseases, Cori's disease, intestinal carbohydrate malabsorption, maltase-, lactase-, sucrase-insufficiency, disorders of the metabolism of fructose, disorders of the metabolism of galactose, galactosaemia, disturbances of pyruvate metabolism, hypolipidemia, hypolipoproteinemia, hyperlipidemia, hyperlipoproteinemia, camitine or camitine acyltransferase deficiency, porphyrias, disturbances of the purine metabolism, lysosomal diseases, metabolic diseases of nerves and nervous systems like gangliosidoses, sphingolipidoses, sulfatidoses, leucodystrophies, Lesch-Nyhan syndrome, dysfunction of the parathyroid glands, pancreatic islet cell dysfunction, carbohydrate and lipid storage myopathies, glycogenoses, myoglobinuria, alkaptonuria, adrenogenital syndrome, ketosis, ketoacidosis, methylmalonaciduria, Morbus Addison, Morbus Conn, Morbus Cushing, Morbus Fabry, Morbus Gaucher, Morbus Hunter, cystic fibrosis, phenylketonuria, thesaurismosis, uricopathia.

The invention relates also to pharmaceutical compositions comprising or consisting of an effective amount of at least one inventive compound, and at least one pharmaceutically acceptable carrier, excipient, binders, disintegrates, glidents, diluents, lubricants, coloring agents, sweetening agents, flavoring agents, preservatives, solvent or the like. The pharmaceutical compositions of the present invention can be prepared in a conventional solid or liquid carrier or diluents and a conventional pharmaceutically-made adjuvant at suitable dosage level in a known way.

According to the invention, the inventive compound or the pharmaceutical composition can be used for the treatment of metabolic diseases, preferably of diabetes mellitus, more preferably of diabetes mellitus type 2, and most preferably to increase the level of insulin release from pancreas cells.

The inventive pharmaceutical composition is formulated to be compatible with its intended route of administration. Administration forms include, for example, pills, tablets, film tablets, coated tablets, capsules, liposomal formulations, micro- and nano-formulations, powders and deposits. Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain the compound according to the present invention. Intravenous and oral applications are preferred forms of administration in the present invention, wherein oral application is particularly preferred.

The present invention also includes the mammalian milk, artificial mammalian milk as well as mammalian milk substitutes as a formulation for oral administration of the inventive compound to newborns, toddlers, and infants, either as pharmaceutical preparations, and/or as dietary food supplements.

The inventive compound can also be administered in form of its pharmaceutically active salts. Suitable pharmaceutically active salts comprise acid addition salts and alkali or earth alkali salts. For instance, sodium, potassium, lithium, magnesium or calcium salts can be obtained.

The pharmaceutical compositions according to the present invention will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersible granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices. The pharmaceutical compositions may be comprised of 5 to 95% by weight of the inventive compound.

As pharmaceutically acceptable carrier, excipient and/or diluents can be used lactose, starch, sucrose, cellulose, magnesium stearate, dicalcium phosphate, calcium sulfate, talc, mannitol, ethyl alcohol (liquid filled capsules).

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations which are intended to be converted, shortly before use, to liquid form preparations for either oral or parenteral administration. Such liquid forms include solutions, suspensions and emulsions.

The inventive compound may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix.

Powders for constitution refer to powder blends containing the active ingredients and suitable diluents which can be suspended in water or juices. One example for such an oral administration form for newborns, toddlers and/or infants is a human breast milk substitute which is produced from milk powder and milk whey powder, optionally and partially substituted with lactose.

Human breast milk is a complex fluid, rich in nutrients and in non-nutritional bioactive components. It contains all of the nutrients needed by the newborn baby. These include the metabolic components (fat, protein, and carbohydrates), water, and the raw materials for tissue growth and development, such as fatty acids, amino acids, minerals, vitamins, and trace elements.

More than 98% of the fat in is in the form of triglycerides. Oleic acid and palmitic acid are the most abundant fatty acids in breastmilk triglycerides, with comparatively high proportions of the essential fatty acids, and linolenic acid, followed by long-chain polyunsaturated fatty acids, such as arachidonic acid and docosahexaenoic acid. These long-chain fatty acids are constituents of brain and neural tissue and are needed in early life for mental and visual development. The lipid component of breast milk is the transport vehicle for fat-soluble micronutrients such as prostaglandins and vitamins A, D, E, and K.

Proteins account for approximately 75 % of the nitrogen-containing compounds in breast milk. Non-protein nitrogen substances include urea, nucleotides, peptides, free amino acids, and DNA. The proteins of breast milk can be divided into two categories: micellar caseins and aqueous whey proteins, present in the ratio of about 40:60. Casein forms micelles of relatively small volume and produces a soft, flocculent curd in the infant's stomach. The major whey proteins are lactalbumin, lactoferrin, secretory IgA, and serum albumin, with a large number of other proteins and peptides present in smaller amounts.

The principal carbohydrate is lactose, a disaccharide produced in the mammary epithelial cell from glucose by a reaction involving lactalbumin.

In addition to the nutritional components, breast milk contains a wealth of bioactive components that have beneficial non-nutritional functions. These include a wide range of specific and non-specific antimicrobial factors; cytokines and anti-inflammatory substances; and hormones, growth modulators, and digestive enzymes, many of which have multiple activities. These components may be of particular importance for young infants because of the immaturity of the host defense and digestive systems early in life.

The artificial mother milk formulations or mother milk substitutes of the present invention are preferably prepared by adding to a mother milk formulation including commercially available mother milk formulations especially in powder form of the compound of the present invention. The inventive compound is preferably added in an amount of 3 - 100 µg compound or per 100 ml (commercially available) mother milk formulation, more preferably in an amount of 5 - 70 µg / 100 ml and most preferably in an amount of 10 - 40 µg /µ100 ml mother milk formulation.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Other preferred pharmaceutical compositions are buffered solutions. The term buffer, buffer system, buffer solution and buffered solution, when used with reference to hydrogen-ion concentration or pH, refers to the ability of a system, particularly an aqueous solution, to resist a change of pH on adding acid or alkali, or on dilution with a solvent. Preferred buffer systems can be selected from the group consisting of formate (pKa=3.75), lactate (pKa=3.86), benzoic acid (pKa=4.2) oxalate (pKa=4.29), fumarate (pKa=4.38), aniline (pKa=4.63), acetate buffer (pKa=4.76), citrate buffer (pKa2=4.76,pKa3=6.4), glutamate buffer (pKa=4.3), phosphate buffer (pKa=7.20), succinate (pKa1=4.93;pKa2=5.62), pyridine (pKa=5.23), phthalate (pKa=5.41); histidine (pKa=6.04), MES (2-(N-morpholino)ethanesulphonic acid; pKa=6.15); maleic acid (pKa=6.26); cacodylate (dimethylarsinate, pKa=6.27), carbonic acid (pKa=6.35), ADA (N-(2-acetamido)imino-diacetic acid (pKa=6.62); PIPES (4-piperazinebis-(ethanesulfonic acid; BIS-TRIS-propane (1,3-bis[tris(hydroxymethyl)methylamino]-propane), pKa=6.80), ethylendiamine (pKa=6.85), ACES 2-[(2-amino-2-oxoethyl)amino]ethanesulphonic acid; pKa=6.9), imidazole (pKa=6.95), MOPS (3-(N-morphin)-propansulfonic acid; pKa=7.20), diethylmalonic acid (pKa=7.2), TES (2-[tris (hydroxymethyl) methyl] amino ethanesulphonic acid; pKa=7.50) and HEPES (N-2-hydroxylethylpiperazin-N-2-ethansulfonic acid; pKa=7.55) buffers or other buffers having a pKa between 3.8 to 7.7.

Preferred is the group of carboxylic acid buffers such as acetate and carboxylic diacid buffers such as fumarate, tartrate and phthalate and carboxylic triacid buffers such as citrate. Another group of preferred buffers is represented by inorganic buffers such as sulfate, borate, carbonate, oxalate, calcium hydroxyde and phosphate buffers. Another group of preferred buffers are nitrogen containing buffers such as imidazole, diethylenediamine, and piperazine.

Also preferred are sulfonic acid buffers such as TES, HEPES, ACES, PIPES, [(2-hydroxy-1,1-bis(hydroxymethyl)ethyl)amino]-1-propanesulfonic acid (TAPS), 4-(2-hydroxyethyl)piperazine-1-propanesulfonic acid (EPPS), 4-Morpholinepropanesulfonic acid (MOPS) and N,N-bis(2-hydroxyethyl)-2-aminoethanesulfonic acid (BES).

Another group of preferred buffers are glycine buffers such as glycine, glycyl-glycine, glycyl-glycyl-glycine, N,N-bis(2-hydroxyethyl)glycine and N-[2-hydroxy-1,1-bis(hydroxymethyl)ethyl]glycine (Tricine).

Preferred are also amino acid buffers such as glycine, alanine, valine, leucine, isoleucine, serine, threonine, phenylalanine, tyrosine, tryptophane, lysine, arginine, histidine, aspartate, glutamate, asparagine, glutamine, cysteine, methionine, proline, 4-hydroxyproline, N,N,N-trimethyllysine, 3-methylhistidine, 5-hydroxylysine, O-phosphoserine, γ-carboxyglutamate, ε-N-acetyllysine, ω-N-methylarginine, citrulline, ornithine and derivatives thereof.

Preferred are the buffers having an effective pH range of from 2.7 to 8.5, and more preferred of from 3.8 to 7.7. The effective pH range for each buffer can be defined as pKa - 1 to pKa + 1, where Ka is the ionization constant for the weak acid in the buffer and pKa = - log K.

Most preferred are buffers suitable for pharmaceutical use e.g. buffers suitable for administration to a patient such as acetate, carbonate, citrate, fumarate, glutamate, lactate, phosphate, phthalate, and succinate buffers. Particularly preferred examples of commonly used pharmaceutical buffers are acetate buffer, citrate buffer, glutamate buffer and phosphate buffer. Also most preferred is the group of carboxylic acid buffers. The term "carboxylic acid buffers" as used herein shall refer to carboxylic mono acid buffers and carboxylic diacid buffers as well as carboxylic triacid buffers. Of course also combinations of buffers, especially of the buffers mentioned herein are useful for the present invention.

Some suitable pharmaceutical buffers are a citrate buffer (preferably at a final formulation concentration of from about 20 to 200 mM, more preferably at a final concentration of from about 30 to 120 mM) or an acetate buffer (preferably at a final formulation concentration of about 20 to 200 mM) or a phosphate buffer (preferably at a final formulation concentration of about 20 to 200 mM).

Techniques for the formulation and administration of the compound of the present invention may be found in "Remington's Pharmaceutical Sciences" Mack Publishing Co., Easton PA. A suitable composition comprising the compound mentioned herein may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A particularly preferred pharmaceutical composition is a lyophilised (freeze-dried) preparation (lyophilisate) suitable for administration by inhalation or for intravenous administration. To prepare the preferred lyophilised preparation the compound of the invention is solubilised in a 4 to 5% (w/v) mannitol solution and the solution is then lyophilised. The mannitol solution can also be prepared in a suitable buffer solution as described above.

Further examples of suitable cryo- / lyoprotectants (otherwise referred to as bulking agents or stabilizers) include thiol-free albumin, immunoglobulins, polyalkyleneoxides (e.g. PEG, polypropylene glycols), trehalose, glucose, sucrose, sorbitol, dextran, maltose, raffinose, stachyose and other saccharides (cf. for instance WO 97/29782), while mannitol is used preferably. These can be used in conventional amounts in conventional lyophilization techniques. Methods of lyophilisation are well known in the art of preparing pharmaceutical formulations.

For administration by inhalation the particle diameter of the lyophilised preparation is preferably between 2 to 5 µm, more preferably between 3 to 4 µm. The lyophilised preparation is particularly suitable for administration using an inhalator, for example the OPTINEB^{®} or VENTA-NEB^{Ⓡ} inhalator (NEBU-TEC, Elsenfeld, Germany). The lyophilised product can be rehydrated in sterile distilled water or any other suitable liquid for inhalation adminstration.

Alternatively for intravenous administration the lyophilised product can be rehydrated in sterile distilled water or any other suitable liquid for intravenous administration.

After rehydration for administration in sterile distilled water or another suitable liquid the lyophilised preparation should have the approximate physiological osmolality of the target tissue for the rehydrated compound preparation i.e. blood for intravenous administration or lung tissue for inhalation administration. Thus it is preferred that the rehydrated formulation is substantially isotonic.

The preferred dosage concentration for either intravenous, oral, or inhalation administration is between 100 to 2000 µmole/ml, and more preferably is between 200 to 800 µmole/ml. These are also the preferred ranges of the compound in the mother milk substitute or artificial mother milk formulation or the pharmaceutical compositions disclosed herein.

Still another aspect of the present invention relates to the use of the inventive compound as a dietary supplement. That dietary supplement is preferably for oral administration and especially but not limited to administration to newborns, toddlers, and/or infants. A dietary supplement is intended to supplement the diet. The "dietary ingredients" in these products may in addition include: vitamins, minerals, herbs or other botanicals, amino acids, and substances such as enzymes, organ tissues, glandulars, and metabolites. Dietary supplements may be manufactured in forms such as tablets, capsules, softgels, gelcaps, liquids, or powders.

The invention further relates to a method for screening for a modulator for treatment of a metabolic disease, the method comprising
a) contacting a test compound with at least one polypeptide selected from the group consisting of SCYL1, ADCK1, GRK5 and CSNK2A1 polypeptide,
b) detecting the binding of said test compound to the SCYL1, ADCK1, GRK5 or CSNK2A1 polypeptide, and
c) determining the activity of the SCYL1, ADCK1, GRK5 or CSNK2A1 polypeptide in the presence of said test compound.

The screening method of the present invention apparently consists of three steps. The term test compound may be any of the potential modulators listed above. The contacting of the test compound with at least one of the polypeptides can happen e.g. in the form of a compound library, in physiological or non-physiological solution, or solid phase systems, however a liquid environment is preferred. The conditions and the time need to be sufficient to allow the test compound to bind to the polypeptide(s). The method is normally carried our in solution at room temperature and at a suitable pH value normally between pH 5 and 9, all parameters which are easily selected by a skilled person.

The polypeptides SCYL1, ADCK1, GRK5 and/or CSNK2A1 can be obtained by purification from primary human cells, cell lines, from cells which have been transfected with expression constructs which contain the nucleic acid sequences encoding one or more of the polypeptides SCYL1, ADCK1, GRK5 and/or CSNK2A1, or by direct chemical synthesis.

The nucleic acid sequences encoding the polypeptides SCYL1, ADCK1, GRK5 and/or CSNK2A1 can be obtained by cloning the relevant genes, amplification of the cDNAs or chemical synthesis of the nucleic sequences. For the expression of the corresponding polypeptides the nucleic acid sequences can be inserted into expression vectors, such as recombinant bacteriophage, plasmid, or cosmid DNA expression vectors.

The term binding refers to an interaction between the test compound and one or more of the polypeptides SCYL1, ADCK1, GRK5 and/or CSNK2A1 or the nucleic acids encoding one or more of the polypeptides SCYL1, ADCK1, GRK5 and/or CSNK2A1. For binding to a protein, the binding interaction is dependent upon the presence of a particular structure of the kinase, e.g. the antigenic determinant or epitope, recognized by the binding molecule. For binding of compounds to nucleic acids, test compounds need to have a complementary sequence to the nucleic acids, or fit into certain secondary or tertiary structures of the nucleic acids.

The binding of the test compounds to the polypeptides or nucleic acids can be checked by any convenient method known in the art. A separation step may be included to separate bound from unbound components. To check whether the test compound has been bound by the polypeptide or nucleic acid, it is advantageous if the test compound is labeled for direct detection (radioactivity, luminescence, fluorescence, optical or electron density etc.) or indirect detection (e.g., epitope tag such as the FLAG, V5 or *myc* epitopes, an enzyme tag such as horseradish peroxidase or luciferase, a transcription product, etc.). The label may be bound to a substrate, to the proteins employed in the assays, or to the candidate pharmacological agent. The binding of a test compound can also be conveniently checked if one of the components is immobilized on a solid substrate. The substrate can be made of a wide variety of materials and in various shapes, e.g. tubes, microtiter plates, microbeads, dipsticks and the like. It is also advantageous if one of the components is modified by biotinylation, so that the components can be immobilized on streptavidin-covered surfaces.

Protein-DNA interactions can be for instance checked by gel shift aka band shift assays aka elektrophoretic mobility shift assays, which is based on the observation that complexes of protein and DNA migrate through a non-denaturing polyacrylamide gel more slowly that a free DNA fragments.

Protein-RNA interactions can be investigated by RNA electrophoretic mobility shift assays which are an in vitro technique used to detect protein-RNA interactions through changes in migration speed during gel electrophoresis. After incubation, the binding reaction is then separated via non-denaturing polyacrylamide gel electrophoresis. Like protein-DNA complexes, a protein-RNA complex migrates more slowly than a free RNA probe through a gel matrix. This causes a migration shift relative to the nonbound RNA probe. Specificity is determined through a competition reaction, where excess unlabeled RNA is incubated in the binding reaction, resulting in a decrease in the shifted signal if the labeled and unlabeled RNA sequences compete for binding of the same protein. Alternatively, the protein-RNA complex may be crosslinked and the reaction run on a denaturing gel. Specificity is determined through visualization of a single shifted band. Traditionally, RNA probes are radioactively labeled for detection, although fluorescent and chemiluminescent detection is also possible. Non-radioactive RNA end-labeling techniques are limited, but more versatile biotin and fluorescent labeling methods are now available. Alternatively, RNA Pull-down assays can be carried out which selectively extract a Protein-RNA complex from a sample. This method has the advantage that several RNAs can be used with the target protein(s), and selectively binding RNAs can be identified. Typically, the RNA pull-down assay takes advantage of high affinity tags, such as biotin or azido-phosphine chemistry. RNA probes can be biotinylated, complexed with a protein from a cell lysate and then purified using agarose or magnetic beads. Alternatively, the protein may be labeled, or the RNA-Protein complex may be isolated using an antibody against the protein of interest. The RNA is then detected by Northern blot or through RT-PCR analysis and the proteins detected by Western blotting or mass spectrometry. Protein-RNA interactions can also be identified by oligonucleotide-targeted RNase H protection assays (RPA), which is a powerful method for detecting RNA and RNA fragments in cell extracts. Unlike Northern blotting or RT-PCR analysis, RPA assays allow greater flexibility in the integrity of target RNA, requiring very short segments for hybridization and detection. RPA assays can also be used to map protein-RNA interactions. In this adaptation of the RPA, RNase H is used to cleave a target RNA molecule at a specific site hybridized with a DNA probe. If a protein is bound to the RNA at the target sequence, it will prevent will block probe hybridization, prevent cleavage by RNase H and indicate a site of interaction between protein and RNA. RNase H requires only a four basepair hybrid with a DNA probe in order to cleave the RNA molecule of interest. Using many small probes allows the entire sequence of RNA to be mapped for sites sites of interaction.

The interactions between peptides and proteins, respectively, can be investigated by various methods, which include, but are not limited to, protein binding microarray, antibody microarrays, protein chips, and a variety of assays, UV-crosslink experiments.

The interactions between nucleic acids can be checked for instance by hybridization, which is based on the annealing of complementary DNA-DNA or DNA-RNA or RNA-RNA-sequences. The nucleotide sequences encoding SCYL1, ADCK1, GRK5 and CSNK2A1 may be labeled by standard methods and added to a sample of nucleic acids to be used as test compounds under conditions suitable for the formation of hybridization complexes. After a suitable incubation period, the sample is washed and the signal is quantified and compared with a standard value. If the amount of signal in the patient sample is significantly 15 altered from that of a comparable control sample, the nucleotide sequences have hybridized with nucleotide sequences in the sample, and the presence of altered levels of nucleotide sequences encoding GRK5 in the sample indicates the presence of the associated disorder. Such assays may also be used to evaluate the efficacy of a particular therapeutic treatment regimen in animal studies, in clinical trials, or in monitoring the treatment of an individual patient. Interactions between nucleic acids can also be investigated by microarrays. A further way of testing the binding between nucleic acids is the use of gel shift assays, in which hybrid molecules are moving slower in a denaturating gels in electrophoresis.

In all methods to identify compounds that modulate (stimulate or inhibit) the expression and kinase activity of the polypeptides of the invention, the expression level and kinase activity are compared to those detected in the absence of the test compound. The present invention is related particularly to the identification of compounds which have inhibitory activity on the kinase activity of the polypeptides of the invention. Consequently, it is particularly the inhibition of expression and activity that is measured.

The inhibition of nucleic acids on the mRNA-level encoding the polypeptides can be checked by investigating the expression of the polypeptides by quantitative methods, e.g. Western blot or enzyme-linked immune-adsorbent assay (ELISA). A way to quantify the protein expression is further the measuring of fusion proteins, wherein the polypeptides of the invention are fused to proteins or protein fragments which are easy to quantify, like fluorescent proteins. The inhibition of DNA and thus the production of mRNA can be checked by mRNA-quantification. Levels of mRNA can be quantitatively measured by Northern blotting. Another way is the reverse transcription quantitative polymerase chain reaction (RT-PCR followed by qPCR). Another way of quantifying mRNA is the use of microarrays, which are, however, more practical if a large set of mRNAs is investigated.

The inhibition of the polypeptides on the protein-level can be investigated by measuring their activity. The determination of the activity of a polypeptide/protein/enzyme depends depends on its specificity. Consequently, the activity of kinases is measured in phosphorylation assays, wherein a substrate is phosphorylated by a kinase. The kinase activity of SCYL1, ADCK1, GRK5 and CSNK2A1 can be detected, for example, by adding ATP having radioactively labeled phosphate to the system containing the polypeptides SCYL1, ADCK1, GRK5 and/or CSNK2A1 and the substrate and measuring the radioactivity of the phosphate attached to the substrate.

According to the invention, the effect of a test compound on the kinase activity of the polypeptides of the invention can be estimated in a system using an insulin-producing cell line, or primary cells, which are or are derived from pancreatic cells. Therein the change of the insulin release level compared to the level without the compound. The release level of insulin can be estimated with the mRNA and protein quantification levels identified above.

The polypeptides of the invention can be used in high-throughput screens to assay test compounds for the ability to modulate the kinase activity. These compounds can be further screened against a functional kinase to determine the effect of the compound on the kinase activity. Further, these compounds can be tested in animal or invertebrate systems to determine activity/effectiveness. Compounds can be identified that activate (agonist) or inactivate (antagonist) the kinase to a desired degree. Further, SCYL1, ADCK1, GRK5 and/or CSNK2A1 can be used to screen a compound for the ability to stimulate or inhibit interaction between the kinase protein and a molecule that normally interacts with the kinase protein, e.g. a substrate or a component of the signal pathway that the kinase protein normally interacts (for example, another kinase). Such assays typically include the steps of combining the kinase protein with a candidate compound under conditions that allow the kinase protein, or fragrnent, to interact with the target molecule, and to detect the formation of a complex between the protein and the target or to detect the biochemical consequence of the interaction with the kinase protein and the target, such as any of the associated effects of signal transduction such as protein phosphorylation, cAMP turnover, and adenylate cyclase activation, etc..

The polypeptides of the invention are also useful in competition binding assays in methods designed to discover compounds that interact with the kinase (e.g. binding partners and/or ligands). Thus, a compound is exposed to a kinase polypeptide under conditions that allow the compound to bind or to otherwise interact with the polypeptide. Soluble kinase polypeptide is also added to the mixture. If the test compound interacts with the soluble kinase polypeptide, it decreases the amount of complex formed or activity from the kinase target. This type of assay is particularly useful in cases in which compounds are sought that interact with specific regions of the kinase.

To facilitate the identification of modulators of the expression and activity of the peptides of the invention, the invention further provides, in a preferred embodiment, a kit comprising
a) the SCYL1, ADCK1, GRK5 and/or CSNK2A1 polypeptides,
   and/or
b) the nucleic acid encoding SCYL1, the nucleic acid encoding ADCK1, the nucleic acid encoding GRK5 and/or the nucleic acid encoding CSNK2A1,
   and
c) a control compound known to affect the insulin production by binding the SCYL1, ADCK1, GRK5 and/or CSNK2A1 polypeptide or the corresponding nucleic acid.

In a further preferred embodiment, the invention provides a kit comprising
a) the SCYL1, ADCK1, GRK5 and/or CSNK2A1 polypeptides,
   and/or
b) the nucleic acid encoding SCYL1, the nucleic acid encoding ADCK1, the nucleic acid encoding GRK5 and/or the nucleic acid encoding CSNK2A1
   and
c) a control compound known to affect the insulin production by binding the SCYL1, ADCK1, GRK5 and/or CSNK2A1 polypeptide or the corresponding nucleic acid,
   and further comprising
d) a cell line with insulin production.

In a further preferred embodiment, the invention provides a kit comprising
a) the SCYL1, ADCK1, GRK5 and CSNK2A1 polypeptides,
   and/or
b) the nucleic acid encoding SCYL1, the nucleic acid encoding ADCK1, the nucleic acid encoding GRK5 and the nucleic acid encoding CSNK2A1
   and
c) a control compound for each of the kinases SCYL1, ADCK1, GRK5 and CSNK2A1 known to affect the insulin production by binding the SCYL1, ADCK1, GRK5 and CSNK2A1 polypeptide or the corresponding nucleic acid.

In a further preferred embodiment, the invention provides a kit comprising
a) the SCYL1, ADCK1, GRK5 and CSNK2A1 polypeptides,
   and/or
b) the nucleic acid encoding SCYL1, the nucleic acid encoding ADCK1, the nucleic acid encoding GRK5 and the nucleic acid encoding CSNK2A1,
   and
c) a control compound for each of the kinases SCYL1, ADCK1, GRK5 and CSNK2A1 known to affect the insulin production by binding the SCYL1, ADCK1, GRK5 and CSNK2A1 polypeptide or the corresponding nucleic acid,
   and further comprising
d) a cell line with insulin production.

In all embodiments of the kit, the control compounds can be any of the test compounds characterized above. A control compound is used as a reference for the binding/inhibitory efficiency of a test compound because it is known for its binding to a chosen polypeptide or the corresponding nucleic acid which encode the chosen polypeptide, thereby inhibiting the activity or the expression of the polypeptides. A chosen control compound refers to the same polypeptide for which inhibitory compounds are tested; e.g. if compounds for the inhibition of SCYL1 are tested, then the control compound is one which inhibits SCYL1.
In a further preferred embodiment, it is particularly preferred it the kit comprises the polypeptides SCYL1, ADCK1, GRK5 and/or their corresponding nucleic acids.

Inventive control compounds that affect the insulin release by binding to the polypeptides of the invention are e.g. Sunatinib and inventive siRNAs or any other compound which has proved to modulate the insulin production.

The invention is further related to a method for treatment of a metabolic disease, preferably diabetes mellitus, more preferably diabetes mellitus type 2, and most preferably for increasing the level of insulin release from pancreas cells comprising:
administering a subject in need thereof a therapeutically effective amount of at least one modulator for:
   a) inhibition or activation of at least one of the tyrosine kinases selected from the group consisting of SCYL1, ADCK1, GRK5 and CSNK2A1, or
   b) inactivation, degradation, downregulation, intercalation or activation of at least one nucleic acid selected from the group consisting of the nucleic acid encoding SCYL1, the nucleic acid encoding ADCK1, the nucleic acid encoding GRK5 and the nucleic acid encoding CSNK2A1.

An inventive compound known to affect the expression and/or activity of the polypeptides of the invention can be used for the treatment of a metabolic disease, preferably diabetes mellitus, more preferably diabetes mellitus type 2, and most preferably for increasing the level of insulin release from pancreas cells by administration of the inventive compound(s) within pharmaceutical compositions as outline above. In a preferred embodiment of the invention, at least two of the polypeptides of the invention are modulated by one or more of the inventive compounds.

The inventive compounds or inventive compositions are according to the invention useful for each single disease of the group of diseases consisting of metabolic diseases.

The influence of the kinases SCYL1, ADCK1, GRK5 and CSNK2A1 on insulin release suggests a particular, but not limited to, utilization of the polypeptides for diagnosis of metabolic diseases, preferably of diabetes mellitus, more preferably of diabetes mellitus type 2.

The embodiments in the description and the following examples are provided by way of illustration of the invention and are not included for the purpose of limiting the invention. The variations and changes of the invention which are obvious to a person skilled in the field and solutions equivalent to embodiments described herein fall within the scope of protection of the patent claims.

**Table 2 Sequence identities of the target genes and target proteins**

| **SeqldNo** | **Sequence Name** | **Gene Accession** |
|---|---|---|
| | | |
| 9 | SCYL1 gene | NM_023912 |
| 10 | GRK5 gene | NM_018869 |
| 11 | ADCK1 gene | NM_028105 |
| 12 | CSNK2a1 gene | NM_007788 |
| 13 | SCYL1 protein | NM_023912 |
| 14 | GRK5 protein | NM_018869 |
| 15 | ADCK1 protein | NM_028105 |
| 16 | CSNK2a1 protein | NM_007788 |

### Examples

### Example 1: siRNA screen in the pancreatic beta cell line beta-TC6:

To identify the kinases, which might be responsible for the elevated insulin release after Sunitinib treatment, we performed a kinome wide siRNA knock-down screen. The effect on the insulin release of each kinase depletion was monitored by using a rat/mouse insulin ELISA. The resulting data were compared to Sunitinib treatment (5 pM) as positive control and correlated to a non-targeting siRNA. Candidate genes were limited by using hierarchical clustering and by proposing a significant in-/decrease of the insulin release by 15%. Depletion of SCY1-like 1 (SCYL1 ), aarF-containing kinase 1 (ADCK1 ), G protein-coupled receptor kinase 5 (GRK5), and casein kinase 2 alpha 1 (Csnk2A1 ) resulted in an increase of the insulin release in beta-TC6 cells compared to the control siRNA, rendering those kinases as potential negative modulators of insulin release. (Fig. 1 ).

### Example 2: Validation of the negative modulators

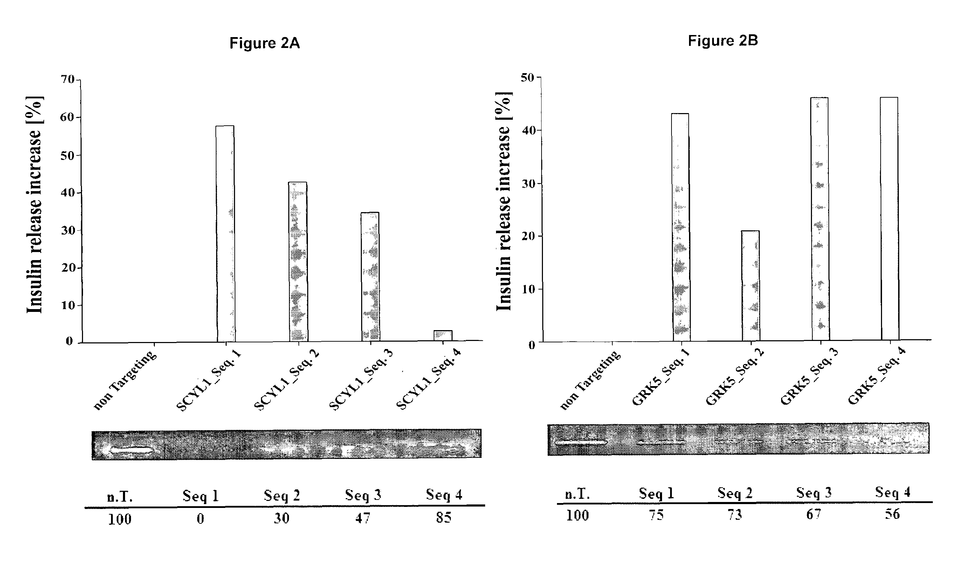

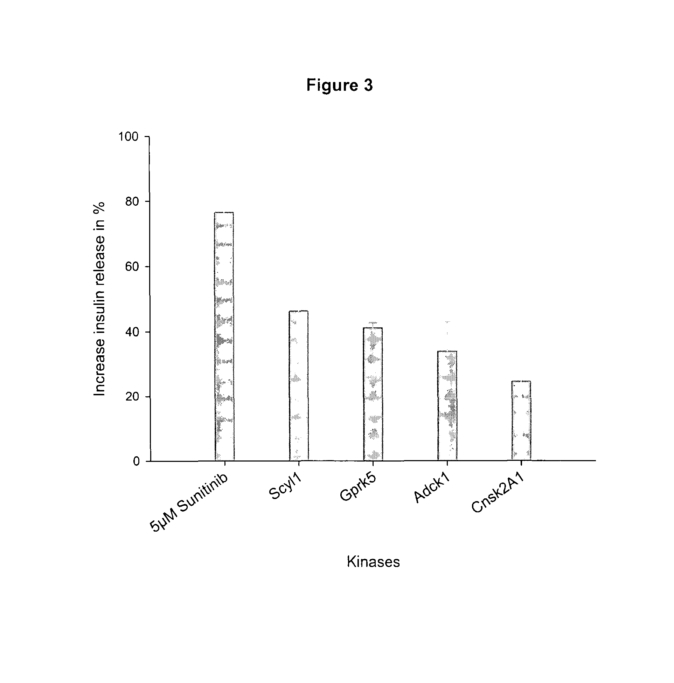

For the validation of the negative modulating kinases SCYL1, GRK5, ADCK1, and CSNK2A1, the target genes were depleted using four different siRNA-sequences each (Fig. 2A-D). The gene-depletion was measured via mRNA-levels after 72 hours while the insulin release was measured using a rat/mouse insulin ELISA after two hours incubation. The depletion of the residual kinases SCYL1, GRK5, ADCK1 and CSNK2A1 led to an increased insulin release with different efficiencies. Furthermore, the insulin release for the kinases inversely correlated with the respective knock-down efficiency of SCYL1, ADCK1 and CSNK2A1 which ranged from 50 to nearly 100 % as estimated by RT-PCR and scanning densitometry. In case of GRK5 where all sequences lead to equal knock-down efficiency, this correlation could not be observed. The increase was highest for the depletion of SCYL1 (46,38 ± 5,51 %), followed by GRK5 (41,23 ± 1,53 %), ADCK1 (33,95 ± 9,02 %) and CSNK2A1 (24,55 ± 2,08 %). Sunitinib was included as a control (Fig. 3). This enhances the role of those kinases in triggering the insulin release.

**Table 1 Sequences of the primers used in RT-PCR for target validation**

| **SeqldNo** | **GeneSymbol** | **Primer** | **GeneAccession** | **Sequence 5' - 3'** |
|---|---|---|---|---|
| 1 | SCYL1 | Fwd | NM_023912 | CGGCGGCGACGATGTGGTTCTTT |
| 2 | SCYL1 | Rev | NM_023912 | CGGCGTTGCCCTGTGCCGAGTA |
| 3 | ADCK1 | Fwd | NM_028105 | CTGACACGGGCAAGGCTGAGATT |
| 4 | ADCK1 | Rev | NM_028105 | GCGCCCTGATACAACACCGAGAC |
| 5 | GRK5 | Fwd | NM_018869 | GCCGGGTGCTGGAGACTGAGGA |
| 6 | GRK5 | Rev | NM_018869 | TGGCGGTTCTGGAGGCTGACTTCT |
| 7 | CSNK2A1 | Fwd | NM_007788 | GGCTGGCCACTTCATCTCTTCTCT |
| 8 | CSNK2A1 | Rev | NM_007788 | TGCGGCCACATCACCCATTAG |

### Example 3: Results double knock-down of the validated kinase

Compared to the Sunitinib treatment, the effect of the single knock-downs was less pronounced suggesting the involvement of multiple kinases in triggering the insulin release. The candidate kinases are widespread in various signalling pathways. To investigate whether the kinases have a redundant or additive effect on insulin release, we performed double-knock-downs for each possible kinase pair. The insulin increase due to the double knock-down was correlated to the single knock-downs as well as to the non-targeting siRNA. Out of 16 kinase pairs, SCYL1 and ADCK1 depletion resulted in the highest Insulin release (90,64 ± 17,32 %), which was equal to the Sunitinib induced insulin release (Fig. 4). For the other kinase pairs, no increased insulin release compared to the single-knock-downs was observed (data not shown).

### Description of the figures

- Figure 1:: Assortment of the positive and negative regulating kinases of the kinome screen. The candidate kinases were limited by using hierarchical clustering and proposing and increased insulin release of 15 % significant. Additionally, the data were correlated to Sunitinib treatment (5 µM) and a non-targeting siRNA as positive and negative controls. The results are shown for ≥n 4 biological independent experiments.

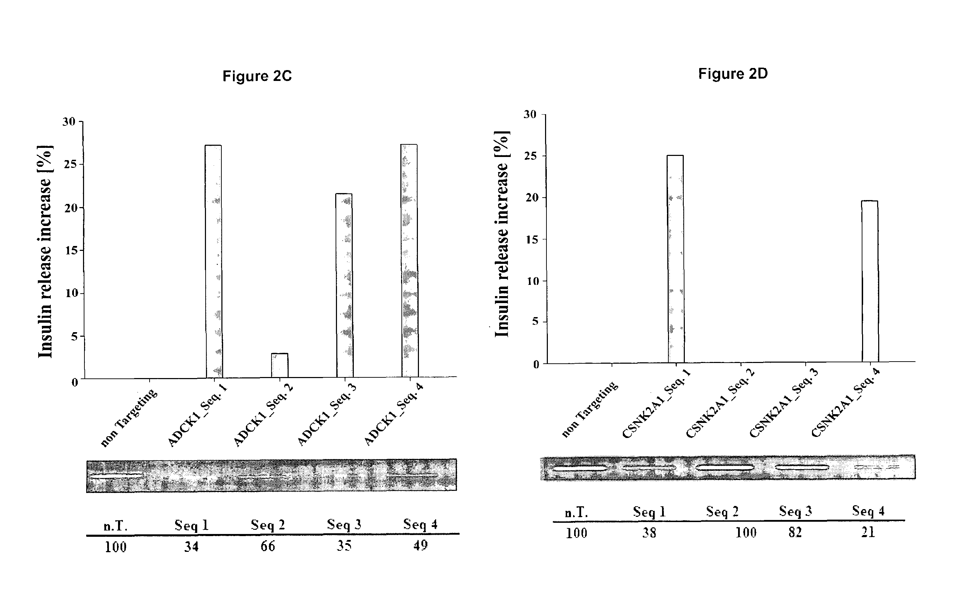

- Figure 2:: Correlation of the insulin release increase to the target depletion for each used siRNA sequence for the kinases SCYL1 (Fig. 2A), GRK5 (Fig. 2B), ADCK1 (Fig. 2C) and CSNK2A1 (Fig. 2D). The insulin release for the kinases (bar chart; upper panel) correlated with their knock-down efficiency, which was monitored by RT-PCR and scanning densitometry (agarose gel; middle panel; Δ depletion; lower panel). The insulin increase as well as the knock-down efficiency is compared to the insulin release or gene depletion of the non-targeting siRNA.
- Figure 3:: Validation of the insulin release increase for the kinases SCYL1, GRK5, ADCK1, and CSNK2A1. SCYL1 showed the most reliable increase in insulin release after gene-depletion with about 46,38 ± 5,51 %, followed by GRK5 (41,23 ± 1,53 %), ADCK1 (33,95 ± 9,02 %), and CSNK2A1 (24,55 ± 2,08 %). The control Sunatinib resulted in 76,52 ± 13 %.
- Figure 4:: Additional increase of the insulin release due to a double knock-down for the kinase pair SCYL1 and ADCK1. The depletion resulted in the highest Insulin release (90,64 ± 17,32 %), which equaled the insulin after Sunitinib treatment. The figure depicts the values of the single knock-down (light grey and grey), the theoretical mathematical value (changeover dark grey to grey) and the real insulin increase after a double knock-down (dark grey).

## Claims

1. A modulator for
a) inhibition of at least one of the protein kinases selected from the group consisting of SCYL1, ADCK1, GRK5 and CSNK2A1, or
b) inactivation, degradation, downregulation, intercalation of at least one nucleic acid selected from the group consisting of the nucleic acid encoding SCYL1, the nucleic acid encoding ADCK1, the nucleic acid encoding GRK5 and the nucleic acid encoding CSNK2A1,
for the treatment of a metabolic disease.

2. The modulator according to claim 1, wherein the metabolic disease is selected from diseases of the carbohydrate metabolism.

3. The modulator according to claim 1 or 2, wherein the disease is diabetes mellitus type 2.

4. The modulator according to claim 1, 2 or 3, wherein said modulator is used for the up-regulation of insulin production and/or release of insulin.

5. The modulator according to any of the claims 1 - 4, wherein the modulator is
a) a small molecule,
b) an RNA molecule,
c) an siRNA molecule, an miRNA molecule, or a precursor thereof,
d) an antisense oligonucleotide,
e) an aptamer
e) a polypeptide,
f) an antibody,
g) a ribozyme, or
h) Sunitinib

6. A pharmaceutical composition comprising the modulator of any of the claims 1 - 5 for the treatment of a metabolic disease.

7. A pharmaceutical composition according to claim 6, wherein the metabolic disease is selected from diseases of the carbohydrate metabolism.

8. A pharmaceutical composition according to claim 6 or 7, wherein the disease is diabetes mellitus type 2.

9. A pharmaceutical composition according to claim 6, 7 or 8 used for the up-regulation of insulin production and/or release of insulin.

10. A method for screening for a modulator for treatment of a metabolic disease, the method comprising
a) contacting a test compound with at least one polypeptide selected from the group consisting of SCYL1, ADCK1, GRK5 and CSNK2A1 polypeptide,
b) detecting the binding of said test compound to the SCYL1, ADCK1, GRK5 or CSNK2A1 polypeptide, and
c) determining the activity of the SCYL1, ADCK1, GRK5 or CSNK2A1 polypeptide in the presence of said test compound.

11. The method according to claim 10, wherein instead of polypeptides nucleic acids encoding the polypeptides are used and the expression rate is determined instead of the activity.

12. The method according to claim 10 or 11, wherein the test compound is RNA or a peptide or an antibody or a kinase inhibitor.

13. A kit comprising
a) the SCYL1, ADCK1, GRK5 and/or CSNK2A1 polypeptides,
b) the nucleic acid encoding SCYL1, the nucleic acid encoding ADCK1, the nucleic acid encoding GRK5 and the nucleic acid encoding CSNK2A1
c) a cell line with insulin production,
d) a control compound known to affect the insulin production by binding the SCYL1, ADCK1, GRK5 and/or CSNK2A1 polypeptide or the corresponding nucleic acid.

14. A method for treatment of a metabolic disease comprising:
administering a subject in need thereof a therapeutically effective amount of at least one modulator for:
a) inhibition or activation of at least one of the tyrosine kinases selected from the group consisting of SCYL1, ADCK1, GRK5 and CSNK2A1, or
b) inactivation, degradation, downregulation, intercalation or activation of at least one nucleic acid selected from the group consisting of the nucleic acid encoding SCYL1, the nucleic acid encoding ADCK1, the nucleic acid encoding GRK5 and the nucleic acid encoding CSNK2A1.
